(19) Europäisches Patentamt
European Patent Office
Office européen des brevets

(11) EP 1 920 370 B1

(12) EUROPEAN PATENT SPECIFICATION

(45) Date of publication and mention of the grant of the patent:
**05.10.2011 Bulletin 2011/40**

(21) Application number: **06783129.7**

(22) Date of filing: **23.08.2006**

(51) Int Cl.:
***A61K 31/282** (2006.01)* ***A61K 31/352** (2006.01)*
***A61P 35/00** (2006.01)* ***A61P 15/10** (2006.01)*

(86) International application number:
**PCT/JP2006/317027**

(87) International publication number:
**WO 2007/026725 (08.03.2007 Gazette 2007/10)**

(54) **MAGNETIC DRUG, MAGNETIC DRUG GUIDANCE SYSTEM AND MAGNETIC DRUG DESIGN METHOD**

MAGNETISCHES MEDIKAMENT, ANLEITUNGSSYSTEM UND VERFAHREN ZUM ENTWURF VON MAGNETISCHEN MEDIKAMENTEN

MEDICAMENT MAGNETIQUE, CIBLAGE MAGNETIQUE DU MEDICAMENT ET PROCEDE DE MISE AU POINT D'UN MEDICAMENT MAGNETIQUE

(84) Designated Contracting States:
**CH DE FR GB LI**

(30) Priority: **31.08.2005 JP 2005251190**
**28.06.2006 JP 2006177971**

(43) Date of publication of application:
**14.05.2008 Bulletin 2008/20**

(73) Proprietors:
- **IHI Corporation**
**Tokyo 135-8710 (JP)**
- **Ishikawa, Yoshihiro**
**Tokyo 160-0022 (JP)**

(72) Inventors:
- **ISHIKAWA, Yoshihiro**
**Tokyo 1600022 (JP)**
- **EGUCHI, Haruki**
**Tokyo 1358710 (JP)**

(74) Representative: **Lamb, Martin John Carstairs et al**
**Marks & Clerk LLP**
**90 Long Acre**
**London**
**WC2E 9RA (GB)**

(56) References cited:
**EP-A1- 0 800 829** **EP-A2- 1 097 711**
**WO-A1-99/64004** **US-A- 4 871 716**

- **ZHANG Y ET AL: "Surface modification of superparamagnetic magnetite nanoparticles and their intracellular uptake" BIOMATERIALS, ELSEVIER SCIENCE PUBLISHERS BV., BARKING, GB, vol. 23, no. 7, 1 April 2002 (2002-04-01), pages 1553-1561, XP004348166 ISSN: 0142-9612**
- **GUPTA A J ET AL: "RECEPTOR-MEDIATED TARGETING OF MAGNETIC NANOPARTICLES USING INSULIN AS A SURFACE LIGAND TO PREVENT ENDOCYTOSIS" IEEE TRANSACTIONS ON NANOBIOSCIENCE, IEEE SERVICE CENTER, PISCATAWAY, NY, US, vol. 2, no. 4, December 2003 (2003-12), pages 255-261, XP009067638 ISSN: 1536-1241**
- **SESTIER C ET AL: "SURFACE MODIFICATION OF SUPERPARAMAGNETIC NANOPARTICLES (FERROFLUID) STUDIED WITH PARTICLE ELECTROPHORESIS: APPLICATION TO THE SPECIFIC TARGETING OF CELLS" ELECTROPHORESIS, WILEY-VCH VERLAG, WEINHEIM, DE, vol. 19, no. 7, June 1998 (1998-06), pages 1220-1226, XP008005573 ISSN: 0173-0835**
- **TORCHILIN V P ET AL: "MAGNETIC SEPHADEX AS A CARRIER FOR ENZYME IMMOBILIZATION AND DRUG TARGETING" JOURNAL OF BIOMEDICAL MATERIALS RESEARCH, WILEY, NEW YORK, NY, US, vol. 19, no. 4, April 1985 (1985-04), pages 461-466, XP001006106 ISSN: 0021-9304**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BHAT S V ET AL: "STRUCTURES AND STEREOCHEMISTRY OF NEW LABDANE DI TERPENOIDS FROM COLEUS-FORSKOHLII" TETRAHEDRON LETTERS, vol. 18, no. 19, 1977, pages 1669-1672, XP002453562 ISSN: 0040-4039
- LECLAIRE ET AL: "A simple access to a forskolin precursor" TETRAHEDRON LETTERS, ELSEVIER, AMSTERDAM, NL, vol. 30, no. 46, 1989, pages 6331-6334, XP022051001 ISSN: 0040-4039
- ROTELLA D P ET AL: "N-3-Substituted Imidazoquinazolinones: Potent and Selective PDE5 Inhibitors as Potential Agents for Treatment of Erectile Dsyfunction" JOURNAL OF MEDICINAL CHEMISTRY, AMERICAN CHEMICAL SOCIETY. WASHINGTON, US, vol. 43, no. 7, 6 April 2000 (2000-04-06), pages 1257-1263, XP002174403 ISSN: 0022-2623
- ROTELLA D P ET AL: "Optimisation of Substiuted N-3-Benzylimidazoquinazolinone Sulfonamides as Potent and Selective PDE5 Inhibitors" JOURNAL OF MEDICINAL CHEMISTRY, AMERICAN CHEMICAL SOCIETY. WASHINGTON, US, vol. 43, no. 26, 28 December 2000 (2000-12-28), pages 5037-5043, XP002174404 ISSN: 0022-2623
- LEOPOLD, WILBUR R. ET AL: "Carcinogenicity of antitumor cis-platinum(II) coordination complexes in the mouse and rat" CANCER RESEARCH , 39(3), 913-18 CODEN: CNREA8; ISSN: 0008-5472, 1979, XP002453563
- TAKAHASHI, IKUO ET AL: "Heat enhances the cytotoxicity of cis-diamminedichloroplatinum(II) and its analogs cis-1,1-cyclobutanedicarboxylato(2R)-2-met hyl-1,4-butanediammineplatinum(II) and cis-diammine (glycolato)platinum in vitro" CANCER CHEMOTHERAPY AND PHARMACOLOGY ( 1993 ), 33(1), 31-5 CODEN: CCPHDZ; ISSN: 0344-5704, January 1994 (1994-01), XP008084375
- MOMBRU A W ET AL: "Multilevel ferromagnetic behavior of room-temperature bulk magnetic graphite" PHYSICAL REVIEW. B, CONDENSED MATTER AND MATERIALS PHYSICS, AMERICAN INSTITUTE OF PHYSICS, WOODBURY, NY, US, vol. 71, no. 10, 1 March 2005 (2005-03-01), pages 100404-1, XP002340721 ISSN: 1098-0121
- HALBREICH A ET AL: "BIOMEDICAL APPLICATIONS OF MAGHEMITE FERROFLUID" BIOCHIMIE, MASSON, PARIS, FR, vol. 80, no. 5/6, 1998, pages 379-390, XP008066522 ISSN: 0300-9084
- ALEXIOU C ET AL: "LOCOREGIONAL CANCER TREATMENT WITH MAGNETIC DRUG TARGETING" CANCER RESEARCH, AMERICAN ASSOCIATION FOR CANCER RESEARCH, BALTIMORE, MD, US, vol. 60, no. 23, 1 December 2000 (2000-12-01), pages 6641-6648, XP001063184 ISSN: 0008-5472
- GUPTA P K ET AL: "MAGNETICALLY CONTROLLED TARGETED MICRO-CARRIER SYSTEMS" LIFE SCIENCES, PERGAMON PRESS, OXFORD, GB, vol. 44, no. 3, 1989, pages 175-186, XP001068739 ISSN: 0024-3205
- PARDO ET AL: "Synthesis and characterization of stable room temperature bulk ferromagnetic graphite" CARBON, ELSEVIER, OXFORD, GB, vol. 44, no. 3, 28 September 2005 (2005-09-28), pages 565-569, XP005172479 ISSN: 0008-6223
- IBRAHIM A ET AL: "New magnetic drug carrier." THE JOURNAL OF PHARMACY AND PHARMACOLOGY JAN 1983, vol. 35, no. 1, January 1983 (1983-01), pages 59-61, XP008081122 ISSN: 0022-3573
- KORTUS, J.: "Electronic structure, magnetic ordering and phonons in molecules and solids" INTERNET ARTICLE, [Online] 9 December 2003 (2003-12-09), pages 1-146, XP002455358 http://deposit.ddb.de/cgi-bin/dokserv?idn=969764359&dok_var=d1&dok_ext=pdf&filename= 969764359.pdf [retrieved on 2007-10-16]


Remarks:

The file contains technical information submitted after the application was filed and not included in this specification

## Description

Field of the Invention

The present invention relates to a magnetic drug derivative and a method of preparing a magnetic drug derivative.
Priority is claimed on Japanese Patent Applications No. 2005-251190, filed August 31, 2005, and No. 2006-177971, filed June 28, 2006.

Description of the Related Art

Generally, drugs administered to the living body reach target sites and cause therapeutic effects by exerting pharmacological effects at the localized target sites. However, there will not be a cure if drugs reach tissues other than the target sites (that is, normal tissues). Consequently, how to guide drugs to the target sites efficiently is important in terms of therapeutic strategy. Such a technology for guiding drugs to the target sites is called drug delivery and research and development thereof have been actively carried out in recent years. These drug delivery methods have at least two merits. One is that a sufficiently high drug concentration is obtained in diseased tissues. This is advantageous because pharmacological effects are achieved only when the drug concentration in the target site is higher than a certain value, and therapeutic effects can not be expected when the concentration is low. Second is that the drug delivery methods guide drugs to diseased tissues only and do not guide drugs to normal tissues unnecessarily. Side effects can thereby be suppressed.
Such drug delivery methods exert their effects most in cancer treatments using anticancer agents. Since most anticancer agents suppress cell growth of cancer cells which are actively dividing, they also suppress cell growth in normal tissues where cells are actively dividing such as, for example, bone marrow, hair-roots, or gastrointestinal mucosa. On this account, side effects such as anemia, hair loss, and vomiting appear in cancer patients who have received administration of anticancer agents. Dosage has to be restricted since these side effects would be heavy burdens on patients and thus, there is a problem in that pharmacological effects of anticancer agents cannot be obtained sufficiently. Furthermore, there is a concern of patients dying due to the side effects in worst cases. Accordingly, it is hoped, that cancer treatments can be carried out efficiently while suppressing the side effects by guiding the anticancer agents until they reach cancer cells with drug delivery methods and allowing the agents to exert their pharmacological effects on cancer cells specifically.
Apart from anticancer agents, for example, application of the drug delivery methods to agents for treating male erectile dysfunction is considered. There are examples of significant systemic hypotension resulting in deaths caused by the use of agents for treating male erectile dysfunction when combined with nitro preparations and thus, it is a problem particularly for males of middle and old age with heart disease. This is because the agents for treating erectile dysfunction do not necessarily concentrate at the target site, act on systemic blood vessels, and thereby increase vasodilation effects of nitro preparations. Accordingly, it is considered that the side effects resulting from the combined use with nitro preparations can be suppressed by guiding the agents for treating male erectile dysfunction until they reach the target site with drug delivery methods and allowing the agents to exert their pharmacological effects on the target site specifically.
As a specific method of drug delivery methods, for example, guidance to the target site using supports (carriers) is being studied and this method is to load drugs onto supports that tend to concentrate in the target site and thereby make the supports transport the drugs to the target site. As supports, use of various types of antibodies, microspheres, or magnetic bodies has been discussed. Among them, magnetic bodies are those that are regarded as particularly hopeful and a method to attach the supports, which are magnetic bodies, to the drugs and make them accumulate in the target site by means of a magnetic field has been examined (for example, refer to the following Patent Document 1). Since this guiding method is easy and simple and makes treatment which targets the target site possible, it is considered to be an effective methods especially for anticancer agents with high cytotoxicity.

Patent Document 1 Japanese Laid-Open Patent Application No. 2001-10978

However, when the supports, which are magnetic bodies, are used as carriers as described above, difficulties in oral administration, the large size of carrier molecules in general, or technical problems in bond strength and affinity with the drug molecules have been pointed out and thus, practical application has been difficult.

SUMMARY OF THE INVENTION

The present invention addresses the abovementioned problems, with an object of realizing a drug delivery system which is capable of solving conventional technical problems and which is easy to put into practical application.
According to a first aspect, the present invention provides a magnetic drug derivative obtainable by making a

non-magnetic organic drug compound magnetic by modification of side chains and/or cross-linking between side chains without using supports made from magnetic bodies said derivative being for use in a method of treatment of male erectile dysfunction in which the derivative is guided to a target site of a body by use of the magnetism of the drug derivative itself, wherein said non-magnetic organic drug compound is a PDE5 inhibitor.

The first aspect further provides a method of preparing a magnetic drug derivative, comprising making a non-magnetic organic drug compound magnetic by modification of side chains and/or cross-linking between side chains without using supports made from magnetic bodies said derivative being for use in a method of treatment of male erectile dysfunction in which the derivative is guided to a target side of a body by use of the magnetism of the drug derivative itself, wherein said non-magnetic organic drug compound is a PDE5 inhibitor.

According to a second aspect, the present invention provides a magnetic drug derivative obtainable by making a non-magnetic drug compound magnetic by modification of side chains and/or cross-linking between side chains without using supports made from magnetic bodies said derivative being for use in a method of treatment of cancer in which the derivative is guided to a target site of a body by use of the magnetism of the drug derivative itself, wherein said non-magnetic drug compound is a metal complex.

The second aspect further provides a method of preparing a magnetic drug derivative, comprising making a non-magnetic drug compound magnetic by modification of side chains and/or cross-linking between side chains without using supports made from magnetic bodies said derivative being for use in a method of treatment of cancer in which the derivative is guided to a target site of a body by use of the magnetism of the drug derivative itself, wherein said non-magnetic drug compound is a metal complex.

In the magnetic drug derivative for use according to the second aspect, said metal complex is preferably a cis geometric isomer with anticancer properties.

In the magnetic drug derivative for use according to the second aspect, said cis geometric isomer is preferably cisplatin.

The method of the first or second aspect preferably further comprises determining whether said magnetic drug derivative is ferromagnetic or ferrimagnetic, based on a spin-charge density distribution obtained by a numerical calculation for said magnetic drug derivative.

The method of the first or second aspect preferably further comprises determining the magnetic strength of said magnetic drug derivative, based on a spin-charge density distribution obtained by a numerical calculation for said magnetic drug derivative.

According to a third aspect, the present invention provides magnetic drug derivative of formula:

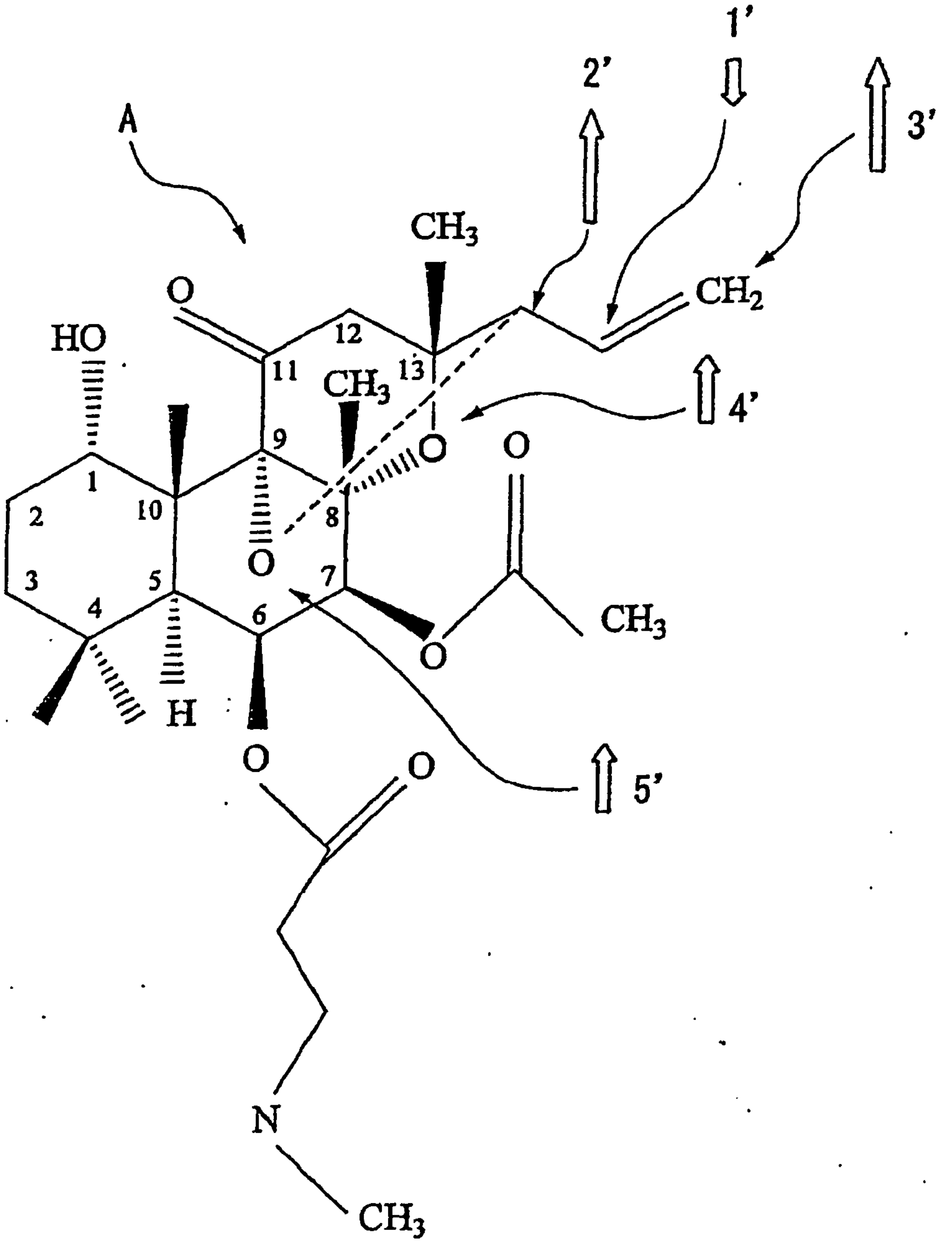

According to a fourth aspect, the present invention provides magnetic drug derivative of formula:

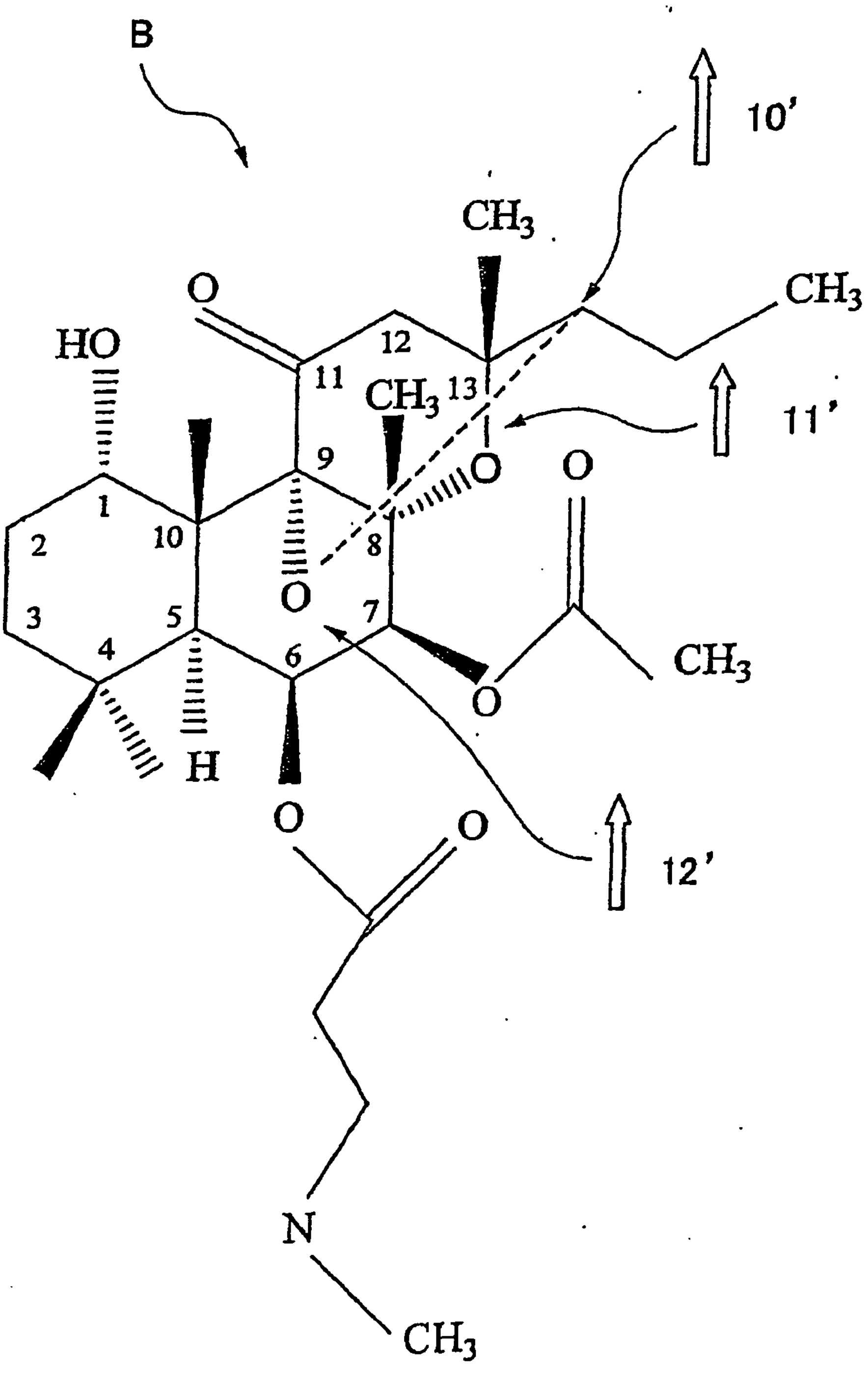

Preferably, the magnetic drug derivative for use according to any of the first or second aspects, or a magnetic drug derivative according to the third or fourth aspects, when administered in a body can be guided to a predetermined target site using the magnetism of the magnetic drug derivative, by a drug guidance system which generates a magnetic field.

Moreover, in the present invention, as a second aspect according to a drug, in the first aspect, the organic compound is forskolin.

In a drug drug guidance system, a drug of any one of the above first to fourth aspects administered to a body can be guided to a predetermined target site using the magnetism of the drug.

In a magnetic detection system, by detecting magnetism of a drug of any one of the above first to fourth aspects administered in a body, the dynamics of the drug can be detected.

A drug according to any one of the first to fourth aspects can be designed according to a drug design method,

comprising: setting with respect to an organic or inorganic compound used as a drug, a molecular model having modified side chains and/or crosslinked side chains; determining whether or not the molecular model is magnetic, from a spin-charge density distribution obtained by a numerical calculation for the molecular model; and then designing the organic compound based on the molecular model that has been determined to be magnetic.

Preferably, it is determined whether the molecular model is ferromagnetic or ferrimagnetic, based on the spin-charge density distribution.

Preferably, the magnetic strength of the molecular model, based on the spin-charge density distribution.

According to the present invention, since drugs themselves will be magnetic, it is possible to guide the drugs to the target sites in the body by use of magnetism of the drugs themselves without using supports made from magnetic bodies as in the conventional cases. As a result, conventional problems such as diffculties in oral administration, the large size of carrier molecules in general, or technical problems in bond strength and affinity with the drug molecules can be resolved. Furthermore, it is possible to realize a drug delivery system which is easy to put into practical application.

BRIEF DESCRIPTION OF THE DRAWINGS

FIG. 1 is a diagram of a basic molecular structural model of forskolin for use in one embodiment of the present invention.
FIG. 2 is a diagram of a molecular structural model of a ferrimagnetic forskolin derivative A in one embodiment of the present invention.
FIG. 3 is a diagram showing a three-dimensional molecular structural model of the forskolin derivative A and its spin-charge density distribution, in one embodiment of the present invention.
FIG. 4 is a diagram of a molecular structural model of a ferromagnetic forskolin derivative B in one embodiment of the present invention.
FIG. 5 is a diagram showing a three-dimensional molecular structural model of the forskolin derivative B and its spin-charge density distribution, in one embodiment of the present invention.
FIG. 6 is a flow chart of a drug design method that may be used to design a drug derivative according to present invention.
FIG. 7A is a diagram of a basic molecular structural model of PDB5 inhibitor with a standard composition and FIG. 7B shows a three-dimensional molecular structure and spin-charge density distribution of the PDE 5 inhibitor with a standard composition for use in one embodiment of the present invention.
FIG. 8A is a diagram of a basic molecular structural model of a derivative of PDE5 inhibitor and FIG. 8B shows a three-dimensional molecular structural model and spin-charge density distribution of the derivative of PDE 5 inhibitor for use in one embodiment of the present invention.
FIG. 9 is a diagram of a basic molecular structural model ofcisplatin in one embodiment of the present invention.
FIG. 10A is a diagram of a basic molecular structural model of a cisplatin derivative (Cis-Pt-a3) and FIG. 10B shows a three-dimensional molecular structural model and spin-charge density distribution of the cisplatin derivative. (Cis-Pt-a3) for use in one embodiment of the present invention.
FIG.11 is an analytical result of spin-charge densities of a cisplatin derivative and a derivative derived by the substitution of platinum of the cisplatin derivative into another metal element in one embodiment of the present invention.
FIG.12 is a diagram of a basic molecular structural model of the cisplatin derivative NK121, and its three-dimensional molecular structural model and spin-charge density distribution for use in one embodiment of the present invention.
FIG. 13 is a diagram showing a hydrolysis process of cisplatin in a living body.
FIG. 14 is a diagram of a three-dimensional molecular structural model and spin-charge density distribution of the cisplatin hydrolysate $[Pt(OH_2)_2(dien)]^{2+}$.

DETAILED DESCRIPTION OF THE INVENTION

Hereunder is a description of one embodiment of the present invention, with reference to the drawings.

[First Embodiment]

Firstly, the first embodiment is described using an organic compound, more specifically, forskolin, as a drug candidate agent.

FIG. 1 is a diagram showing a basic molecular structural model of forskolin. In this drawing, $R_6$, $R_7$, and $R_{13}$ show positions bonded with an atom or a molecule for modifying a side chain of forskolin. Depending on the type of atom or molecule bonded to these positions; the physical property of forskolin varies. In this drawing, one having H

bonded to $R_6$, $CH_3$ bonded to $R_7$, and CH=$CH_2$ bonded to $R_{13}$ is naturally occurring forskolin, and one having the side chain structure changed artificially, that is, forskolin produced by changing the atom or molecule for modifying $R_6$, $R_7$, and $R_{13}$, is called a forskolin derivative. In FIG. 1, $C_1$ to $C_{13}$ represent a carbon atom (C).

FIG. 2 is a diagram showing a basic molecular structural model of a magnetic (ferrimagnetic) forskolin derivative A. As shown in this drawing, the forskolin derivative A is one where $R_6$ of the abovementioned naturally occurring forskolin is changed into $COCH_2CH_2NCH_3$, $R_7$ is $CH_3$, $R_{13}$ is changed into CH-CH=$CH_2$, and the oxygen atom (O) bonded to $C_9$ and the carbon atom bonded to $C_{13}$ are crosslinked.

FIG. 3 shows a three-dimensional molecular structure of the forskolin derivative A and its spin-charge density distribution obtained by a computer simulation based on a well-known first principle molecular dynamics method. In FIG. 3, a region 1 shows a downward spin-charge density, and regions 2 to 5 show upward spin-charge densities. Therefore, as shown in FIG. 2, since a downward spin state 1' and upward spin states 2' to 5' are mixed in the forskolin derivative A, it is found to be a ferrimagnetic body.

On the other hand, FIG. 4 is a diagram showing a basic molecular structural model of a magnetic (ferromagnetic) forskolin derivative B. As shown in this drawing, the forskolin derivative B is one where $R_6$ of the abovementioned naturally occurring forskolin is changed into $COCH_2CH_2NCH_3$, $R_7$ is $CH_3$, $R_{13}$ is changed into CH-$CH_2$-$CH_3$, and the oxygen atom bonded to $C_9$ and the carbon atom bonded to $C_{13}$ are crosslinked.

Similarly to the above, FIG. 5 shows a three-dimensional molecular structure of the forskolin derivative B and its spin-charge density distribution obtained by a computer simulation based on the first principle molecular dynamics method. In FIG. 5, regions 10 to 12 show upward spin-charge densities. Therefore, as shown in FIG. 4, since only upward spin states 10' to 12' are present in the forskolin derivative B, it is found to be a ferromagnetic body.

In this manner, by modifying the side chains of forskolin with predetermined atoms or molecules, and crosslinking between side chains present in predetermined positions, a magnetic forskolin derivative, that is, a drug, can be produced. Hereunder is a description of a design method for such a magnetic drug. FIG. 6 is a flow chart showing a processing procedure of the present drug design method. The processing described hereunder is performed in a computer simulation based on the first principle molecular dynamics method.

Firstly, since there are more than 200 types of forskolin derivatives used as drugs, a forskolin derivative serving as an evaluation target is selected from among these, and its chemical formula is input into the computer simulation (step S1). Here, a case where the abovementioned forskolin derivative A is selected as the forskolin derivative is assumed and described hereunder.

Subsequently, based on the chemical formula of the forskolin derivative A, initial values of upward spin (spin up) wave function $\Phi_\uparrow(r)$, downward spin (spin down) wave-function $\Phi_\downarrow(r)$, spin-up effective potential $V_\uparrow(r)$, spin-down effective potential $V_\downarrow(r)$, spin-up charge density $p_\uparrow(r)$, and spin-down charge density $p_\downarrow(r)$ are set (steep, S2). Here r is a variable showing the coordinates in the three-dimensional spate.

In a case where the respective atoms constituting the forskolin derivative A are present as an isolated atom in the three-dimensional space, the spin-up wave functions $\Phi_\uparrow(r)$ are obtained for each of the respective atoms. The initial value of the spin-up wave function $\Phi_\uparrow(r)$ is the sum of all the spin-up wave functions $\Phi_\uparrow(r)$ that have been obtained in such a manner. Similarly, the initial value of the spin-down wave function $\Phi_\downarrow(r)$ is the sum of all the spin-down wave functions $\Phi_\downarrow(r)$ obtained for each of the respective atoms, in a case where the respective atoms are present as an isolated atom in the three-dimensional space. Moreover, based on the spin-up wave functions $\Phi_\uparrow(r)$ in a case where the respective atoms constituting the forskolin derivative A are present as an isolated atom in the three-dimensional space, the spin-up effective potentials $V_\uparrow(r)$ are obtained for each of the respective atoms. The initial value of the spin-up effective potential $V_\uparrow(r)$ is the sum of all the spin-up effective potentials $V_\uparrow(r)$ that have been obtained for each of the respective atoms. Similarly, the initial value of the effective potential $V_\downarrow(r)$ is the sum of all the spin-down effective potentials $V_\downarrow(r)$ obtained for each of the respective atoms based on the spin-down wave functions $\Phi_\downarrow(r)$ in a case where the respective atoms are present as an isolated atom in the three-dimensional space. Moreover, the initial value of the spin-up charge density $\rho_\uparrow(r)$ is obtained by substituting the spin-up wave functions $\Phi_\uparrow(r)$ that have been obtained for each of the respective atoms as mentioned above, into the following operational expression (1). Moreover, the initial value of the spin-down charge density $\rho_\downarrow(r)$ is obtained by substituting the spin-down wave functions $\Phi_\downarrow(r)$ that have been obtained for each of the respective atoms, into the following operational expression (2). In the following operational expression (1), $\Phi_\uparrow^*(r)$ is a conjugate complex number of the spin-up wave function $\Phi_\uparrow(r)$. In the following operational expression (2), $\Phi_\downarrow^*(r)$ is a conjugate complex number of the spin-down wave function $\Phi_\downarrow(r)$.

[Equation 1]

$$\rho_\uparrow(r) = \sum \Phi_\uparrow{}^*(r)\Phi_\uparrow(r) \quad (1)$$

$$\rho_\downarrow(r) = \sum \Phi_\downarrow{}^*(r)\Phi_\downarrow(r) \quad (2)$$

Next, based on the initial values of the spin-up effective potential $V_\uparrow(r)$ and the spin-down effective potential $V_\downarrow(r)$, and the initial values of the spin-up charge density $\rho_\uparrow(r)$ and the spin-down charge density $\rho_\downarrow(r)$, the following Kohn-Sham equations (3) and (4) are solved, so as to calculate the spin-up wave function $\Phi_\uparrow(r)$, the spin-down wave function $\Phi_\downarrow(r)$, the spin-up energy eigenvalue $\varepsilon_\uparrow$, and the spin-down energy eigenvalue $\varepsilon_\downarrow$ of the forskolin derivative A (step S3).

[Equation 2]

$$\left[-\frac{1}{2}\nabla^2 + V_\uparrow\{r, \rho_\uparrow(r)\}\right]\Phi_\uparrow(r) = \varepsilon_\uparrow\Phi_\uparrow(r) \quad (3)$$

$$\left[-\frac{1}{2}\nabla^2 + V_\downarrow\{r, \rho_\downarrow(r)\}\right]\Phi_\downarrow(r) = \varepsilon_\downarrow\Phi_\downarrow(r) \quad (4)$$

Then, based on the spin-up wave function $\Phi_\uparrow(r)$ and the spin-down wave function $\Phi_\downarrow(r)$ of the forskolin derivative A obtained in step S3, the spin-up charge density $\rho_\uparrow(r)$, the spin-down charge density $\rho_\downarrow(r)$, the spin-up effective potential $V_\uparrow(r)$, and the spin-down effective potential $V_\downarrow(r)$ of the forskolin derivative A are calculated (step S4). It is then determined whether or not these spin-up charge density $\rho_\uparrow(r)$ and spin-down charge density $\rho_\downarrow(r)$ are the same as the previous values of the spin-up charge density $\rho_\uparrow(r)$ and the spin-down charge density $\rho_\downarrow(r)$, which are the initial values in this case (step S5). In this step S5, if it is determined "NO", that is, the previous values (initial values) of the spin-up charge density $\rho_\uparrow(r)$ and the spin-down charge density $\rho_\downarrow(r)$ are not the same as the present values obtained in step S4, then the spin-up effective potential $V_\uparrow(r)$, the spin-down effective potential $V_\downarrow(r)$, the spin-up charge density $\rho_\uparrow(r)$, and the spin-down charge density $\rho_\downarrow(r)$ obtained in step S4 are set as new initial values (step S6). Then the flow proceeds to step S3, and the Kohn-Sham equations (3) and (4) are solved again, so as to calculate a new spin-up wave function $\Phi_\uparrow(r)$, spin-down wave function $\Phi_\downarrow(r)$, spin-up energy eigenvalue $\varepsilon_\uparrow$, and spin-down energy eigenvalue $\varepsilon_\downarrow$. That is, in step S5, the processing from steps S3 to S6 is repeated until the previous values of the spin-up charge density $\rho_\uparrow(r)$ and the spin-down charge density $\rho_\downarrow(r)$ become equal to the present values, to thereby obtain the spin-up wave function $\Phi_\uparrow(r)$, the spin-down wave function $\Phi_\downarrow(r)$, the spin-up energy eigenvalue $\varepsilon_\uparrow$, and the spin-down energy eigenvalue $\varepsilon_\downarrow$ which satisfy the Kohn-Sham equations (3) and (4).

On the other hand, in step S5, if it is determined "YES", that is, the previous values of the spin-up charge density $\rho_\uparrow(r)$ and the spin-down charge density $\rho_\downarrow(r)$ are the same as the present values, then as described above, an interatomic force acting between respective atoms is calculated, based on the spin-up wave function $\Phi_\uparrow(r)$, the spin-down wave function $\Phi_\downarrow(r)$, the spin-up energy eigenvalue $\varepsilon_\uparrow$, and the spin-down energy eigenvalue $\varepsilon_\downarrow$ which satisfy the Kohn-Sham equations (3) and (4), and the structure of the forskolin derivative A is optimized (step S7). That is, the spin-up wave function $\Phi_\uparrow(r)$, the spin-down wave function $\Phi_\downarrow(r)$, and so forth that have been obtained by repeating steps S3 to S6, are merely the optimum values in a model on a two-dimensional plane as shown in FIG. 2, and in practice it is necessary to consider the structure of the forskolin derivative A in the three-dimensional space.

Specifically, in step S7, the respective atoms constituting the forskolin derivative A are moved for a predetermined distance in an optimum direction assumed from the spin-up wave function $\Phi_\uparrow(r)$ and the spin-down wave function $\Phi_\downarrow(r)$, in the three-dimensional space, and an interatomic force acting between the respective atoms at this time is calculated. If the interatomic force at this time becomes 0 and the respective atoms are not moved, it can be determined that the structure of the forskolin derivative-A is optimized. Therefore, the interatomic force acting between the respective atoms after the movement is calculated, and it is determined whether or not the interatomic force becomes 0 (step S8). In this step S8, if it is determined "NO", that is, the interatomic force is not 0 and the structure is not optimized, then the spin-up wave functions $\Phi_\uparrow(r)$ and the spin-down wave functions $\Phi_\downarrow(r)$ in the structures of the respective atoms after the

movement are obtained. Then, the spin-up effective potential $V_{\uparrow}(r)$, the spin-down effective potential $V_{\downarrow}(r)$, the spin-up charge density $\rho_{\uparrow}(r)$, and the spin-down charge density $\rho_{\downarrow}(r)$ obtained from the spin-up wave function $\Phi_{\uparrow}(r)$ and the spin-down wave function $\Phi_{\downarrow}(r)$ are set as new initial values (step S9), and the processing from steps S3 to S8 is repeated. Here, the reason the flow returns to step S3 is that the spin-up wave function $\Phi_{\uparrow}(r)$ and the spin-down wave function $\Phi_{\downarrow}(r)$ are changed according to the structural change of the respective atoms after the movement. Moreover, the structures of the respective atoms after the movement are memorized, and when step S7 is performed again, the respective atoms are moved again for a predetermined distance from the previous structure.

When the structure of such a forskolin derivative A is optimized, then as shown in FIG. 2, the three-dimensional structure is forcibly altered so as to crosslink the oxygen atom bonded to $C_9$ and the carbon atom bonded to $C_{13}$. The atoms selected for such a crosslinking can be optionally changed.

On the other hand, in this step S8, if it is determined "YES", that is, the interatomic force acting between the respective atoms becomes 0 and the structure of the forskolin derivative A is optimized, then the spin-charge density distribution as shown in FIG. 3 is obtained, based on the spin-up wave function $\Phi_{\uparrow}(r)$ and the spin-down wave function $\Phi_{\downarrow}(r)$ in the optimized structure (step S10).

Here, depending on the forskolin derivative selected as the evaluation target, the spin-charge density distribution such as regions 1 to 5 shown in FIG. 3 is not generated, or if the spin-charge density distribution is generated, regions having only a very small amount of spin-charge density (that is magnetic strength) are present. Such a forskolin derivative can not be determined to be magnetic. Consequently, based on the spin-charge density distribution, firstly it is determined whether or not the forskolin derivative selected as the evaluation target is magnetic (step S11).

In step S11, if it is determined "NO", that is, the forskolin derivative selected as the evaluation target is not magnetic, the flow proceeds to step S1, and another forskolin derivative is newly selected and the magnetism is evaluated again. On the other hand, in step S 11, if it is determined "YES", that is, the forskolin derivative selected as the evaluation target is magnetic, then it is determined whether it is ferromagnetic or ferrimagnetic, based on the spin-charge density distribution (step S 12). As described above, since the spin-charge density distribution shows the distribution of the spin-up charge density and the spin-down charge density, if these spin-up charge density and spin-down charge density are mixed, it can be determined to be ferrimagnetic. If only one of the spin-up charge density and the spin-down charge density is present, it can be determined to be ferromagnetic.

As shown in FIG. 3, in the forskolin derivative A, since the spin-up charge densities (regions 2 to 5) and the spin-down charge density (region 1) are mixed, it is determined to be a ferrimagnetic forskolin derivative (step S13). On the other hand, for example, if the selected forskolin derivative is the forskolin derivative B, as shown in FIG. 5, only the spin-up charge densities (regions 10 to 12) are present. Therefore it is determined to be a ferromagnetic forskolin derivative (step S 14). It is also possible to obtain the magnetic strength based on the spin-charge density distribution.

As described above, according to the present drug design method, the magnetism of a forskolin derivative having side chains modified with various atoms or molecules, and side chains optionally crosslinked can be determined. Moreover, by producing a forskolin derivative based on a molecular model determined to be magnetic, a magnetic drug can be manufactured. Therefore, it is possible to guide the drugs to the target sites in the body by use of magnetism of the drugs themselves without using supports (carriers) made from magnetic bodies as in the conventional cases. As a result, conventional problems such as difficulties in oral administration, the large size of carrier molecules in general, or technical problems in bond strength and affinity with the drug molecules can be resolved. Furthermore, it is possible to realize a drug delivery system which is easy to put into practical application.

In the above first embodiment, in both the forskolin derivatives A and B, the three-dimensional structure is forcibly altered so as to crosslink the oxygen atom bonded to $C_9$ and the carbon atom bonded to $C_{13}$. However, it is not limited to this, and other atoms may be selected to be crosslinked. Moreover, by not performing crosslinking, but by simply changing an atom or a molecule for modifying the side chain, it may be determined to be magnetic or not.

Moreover, in the above first embodiment, forskolin is used as an organic compound for description. However, it is not limited to this, and other organic compounds may be used. Hereunder is a description of, as another organic compound, a composition effective in treatments of male erectile dysfunction, more specifically, a composition inhibiting the generation of phosphodiesterase 5 (PDE 5), which hereinafter will be referred to as "PDE 5 inhibitor". Drugs having this PDE 5 inhibitor as an active ingredient are used as remedies for male erectile dysfunction such as so-called Viagra®.

FIG. 7A is a diagram of a basic molecular structural model of PDE5 inhibitor with a standard composition and FIG. 7B shows a three-dimensional molecular structure and spin-charge density distribution of the PDE 5 inhibitor with a standard composition that are obtained by a computer simulation in the abovementioned drug design method. On the other hand, FIG. 8A is a diagram of a basic molecular structural model of a PDE 5 inhibitor derivative derived by subjecting the PDE 5 inhibitor with a standard composition to side chain modifications. FIG. 8B shows a three-dimensional molecular structure and spin-charge density distribution of the PDE 5 inhibitor derivative obtained by the abovementioned computer simulation. In FIG. 8B, the regions 20 to 23 show upward spin-charge densities, and the regions 24 to 26 show downward spin-charge densities. Therefore, the PDE 5 inhibitor derivative is a ferrimagnetic body where the upward spin states 20' to 23' and the downward spin states 24' to 26' coexist as shown in FIG. 8A.

That is, as shown in these FIGS. 7 and 8, although the PDE 5 inhibitor with a standard composition is not magnetic, the PDE 5 inhibitor derivative which is generated by side chain modification is confirmed to be magnetic. Therefore, by using a therapeutic agent for male erectile dysfunction, which has such a magnetic PDE 5 inhibitor derivative as an active ingredient, pharmacological effects of the drug can be brought out specifically in the target site and the occurrence of side effects due to the combined use with the nitro preparations can be suppressed.

[Second Embodiment]

Next, a second embodiment is described using an inorganic compound, more specifically, cisplatin as an anticancer agent. Cisplatin is a metal complex (platinum complex) and classified as a platinum preparation among the anticancer agents.

FIG. 9 is a diagram of a basic molecular structural model of cisplatin with a standard composition. Using the computer simulation in the drug design method described in the first embodiment, this cisplatin with a standard composition is confirmed to be non-magnetic. On the other hand, FIG. 10A is a diagram of a basic molecular structural model of a cisplatin derivative (Cis-Pt-a3), which is derived by subjecting the cisplatin with a standard composition to side chain modifications. Additionally, FIG. 10B shows a three-dimensional molecular structure and spin-charge density distribution of the cisplatin derivative (Cis-Pt-a3) obtained by the abovementioned computer simulation.

In FIG. 10B, the regions 30 to 32 show upward spin-charge densities. Therefore, the cisplatin derivative (Cis-Pt-a3) is found to be a ferromagnetic body where the upward spin states 30' to 32' exist as shown in FIG. 10A. That is, using the computer simulation in the present drug design method, the cisplatin derivative (Cis-Pt-a3) is confirmed to be magnetic. Therefore, by using an anticancer agent, which has such a magnetic cisplatin derivative (Cis-Pt-a3) as an active ingredient, pharmacological effects of the drug can be brought out specifically in the cancer tissues and the occurrence of side effects can be suppressed.

The stronger the magnetism of a drug, the more efficiently the drug can be guided to the target site, and thus, a greater increase in pharmacological effects and suppression of side effects can be expected. Accordingly, the present inventors carried out an analysis of magnetic strength for various cisplatin derivatives using the computer simulation in the present drug design method. The analytical results are described below. Since the magnetic strength is in a linear relationship with the spin-charge density, the spin-charge densities in various cisplatin derivatives are analyzed in the present embodiment.

Firstly, as a reference, particles having a total number of 101 atoms and which were approximately 8A on a side were cut out from a magnetite ($Fe_3O_4$) crystal and were set as the molecular models, and after electronic states and structures were optimized by the abovementioned computer simulation, the analysis of spin-charge densities was performed. Then, by adopting the spin-charge density of the abovementioned magnetite particles as the standard, the analysis of spin-charge densities for various cisplatin derivatives was similarly carried out.

Furthermore, in addition to the cisplatin derivatives, various derivatives where platinum (Pt) of the cisplatin derivatives was substituted by palladium (Pd), rhodium (Rh), iridium (Ir), gold (Au), nickel (Ni), silver (Ag), copper (Cu), or cobalt (Co) were similarly analyzed for their spin-charge densities. The derivatives generated by the substitution of platinum in the cisplatin derivatives with the abovementioned metal elements, as described above, are known to have effects in inhibiting the replication of DNA which is accompanied with the propagation of cancer cells, similarly to cisplatin or cisplatin derivatives.

FIG. 11 shows the analytical results of spin-charge densities of various cisplatin derivatives and of various derivatives where platinum (Pt) of the cisplatin derivatives was substituted by palladium (Pd), rhodium (Rh), iridium (Ir), gold (Au), nickel (Ni), silver (Ag), copper (Cu), or cobalt (Co), when the spin-charge density of the magnetite particles was standardized to "1".

As shown in FIG. 11, among the cisplatin derivatives, it was found that NK121 had approximately 60% of spin-charge density compared to that of the magnetite particles and is effective as a magnetic drug compared to other cisplatin derivatives. This cisplatin derivative NK121 is one which once managed to reach clinical development after a safety test. However, since the anticancer effect thereof was comparable to that of cisplatin, it was determined to have no merits surpassing cisplatin and the development thereof was suspended. Therefore, if this cisplatin derivative NK121 is taken and the guidance of the drug to the target site by means of a magnetic field is performed, drug effects would increase and side effects can also be suppressed to a large extent. FIG. 12 shows a diagram of a basic molecular structural model of the cisplatin derivative NK121. As shown in this diagram, the cisplatin derivative NK121 is a ferromagnetic body where the upward spin states 40' to 42' exist.

Moreover, the derivatives where platinum (Pt) of the cisplatin derivatives was substituted by palladium (Pd) were also confirmed to have spin-charge densities to some extent and thus, were magnetic bodies. In addition, among the derivatives where platinum (Pt) of the cisplatin derivatives was substituted by rhodium (Rh), Cis-Rh-a3 was found to have approximately 50% of spin-charge density compared to that of the magnetite particles and was effective as a magnetic drug. Further, the derivatives where platinum (Pt) of the cisplatin derivatives was substituted by iridium (Ir)

were confirmed to have considerably small spin-charge densities and not many effects as magnetic drugs. Additionally, the derivatives where platinum (Pt) of the cisplatin derivatives was substitute by gold (Au) were also confirmed to have spin-charge densities to some extent and were magnetic bodies.

Moreover, the derivatives where platinum (Pt) of the cisplatin derivatives was substituted by nickel (Ni) generally had approximately 50% of spin-charge densities compared to those of the magnetite particles and were found to be effective as magnetic drugs. Additionally, the derivatives where platinum (Pt) of the cisplatin derivatives was substituted by silver (Ag) were also confirmed to have spin-charge densities to some extent and were magnetic bodies. In addition, the derivatives where platinum (Pt) of the cisplatin derivatives was substituted by copper (Cu) were also confirmed to have spin-charge densities to some extent and were magnetic bodies. Furthermore, it was found that the derivatives where platinum (Pt) of the cisplatin derivatives was substituted by cobalt (Co) had, among higher ones thereof, approximately 95% of spin-charge densities compared to those of the magnetite particles, and also, generally had considerably high spin-charge densities, and were highly effective as magnetic drugs.

As described so far, according to the drug design method in the present embodiment, not only with the drugs comprising organic compounds but also with those comprising inorganic compounds, it is possible to analyze whether they are magnetic or not from molecular models thereof. Moreover, by examining drugs with high magnetic strength (that is, with high drug effects) in advance, it will become possible to design effective drugs with a considerably high efficiency.

The above-described cisplatin derivatives and the derivatives where platinum of the cisplatin derivatives was substituted by other metal elements are cis geometric isomers. Such cis geometric isomers are used as anticancer agents since they have higher effects at inhibiting the replication of DNA which is accompanied with the propagation of cancer cells than those in trans geometric isomers. However, according to the drug design method in the present embodiment, targeted drugs can be analyzed whether they are magnetic or not, not only when they are cis geometric isomers of anticancer agents or the like, but also when they are the metal complexes composed of trans geometric isomers or when they are other inorganic compounds. Therefore, it is also possible to design magnetic drugs comprising the metal complexes composed of trans geometric isomers, or other inorganic compounds.

Next is a description of a guidance system for guiding the abovementioned magnetic drug to a target site.

This guidance system may be any system as long as it generates a magnetic field, and various forms of systems can be considered. For example, as an example, application of magnetic resonance imaging (MRI) is considered, and the construction may be such that a magnetic field is irradiated to the human body and the magnetic field is controlled so as to guide the drug to the target site. Moreover, for example, a magnetic material such as a magnet may be adhered onto the skin surface of the target site. As a result, the drug that has reached the vicinity of the target site is guided to the target site, and stays specifically at the target site, causing no side effects to other normal cells. According to the above guidance system, it is possible to selectively and specifically guide a magnetic drug to the target site.

Furthermore, using the magnetism of the drugs administered in a body, it is also possible to examine the dynamics of the drugs in the body. More specifically, using a magnetic drug as a tracer, the dynamics of the drug in the body are examined by tracing the magnetism generated from the drug with a magnetic detector. With such a magnetic detector, it is possible to examine the dynamics of drugs in the body such as the time taken for the drugs to reach the target sites after being administered in the body and thus, the present invention can contribute to research and development of drugs.

It is known that the cisplatin with a standard composition shown in FIG. 9 is hydrolyzed when administered in the body by the hydrolysis process represented by the reactions 1 to 3 shown in FIG. 13, and finally generates the hydrolysate of cisplatin $[Pt(OH_2)_2(dien)]^{2+}$. As mentioned above, the cisplatin with a standard composition shown in FIG. 9 is not magnetic. However, the present inventors discovered that, based on the present drug design method, this hydrolysate of cisplatin $[Pt(OH_2)_2(dien)]^{2+}$ is magnetic. FIG. 14 shows a three-dimensional molecular structure and spin-charge density distribution of the cisplatin hydrolysate $[Pt(OH_2)_2(dien)]^{2+}$. As shown in this diagram, since the cisplatin hydrolysate $[Pt(OH_2)_2(dien)]^{2+}$ has regions 50 and 51 with upward spin-charge densities, it is found to be a ferromagnetic body.

Therefore, even with the cisplatin with a standard composition, since it is magnetic after being administered in the body, it can be guided to the target site by the abovementioned guidance system and it is also possible to examine the dynamics thereof in the body with a magnetic detector.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

**Claims**

1. A magnetic drug derivative obtainable by making a non-magnetic organic drug compound magnetic by modification of side chains and/or cross-linking between side chains without using supports made from magnetic bodies said derivative being for use in a method of treatment of male erectile dysfunction in which the derivative is guided to a target site of a body by use of the magnetism of the drug derivative itself, wherein said non-magnetic organic drug compound is a PDE5 inhibitor.

2. A magnetic drug derivative obtainable by making a non-magnetic drug compound magnetic by modification of side chains and/or cross-linking between side chains without using supports made from magnetic bodies said derivative being for use in a method of treatment of cancer in which the derivative is guided to a target site of a body by use of the magnetism of the drug derivative itself, wherein said non-magnetic drug compound is a metal complex.

3. A magnetic drug derivative for use according to claim 2, wherein said metal complex is a cis geometric isomer with anticancer properties.

4. A magnetic drug derivative for use according to claim 3, wherein said cis geometric isomer is cisplatin.

5. A magnetic drug derivative of formula:

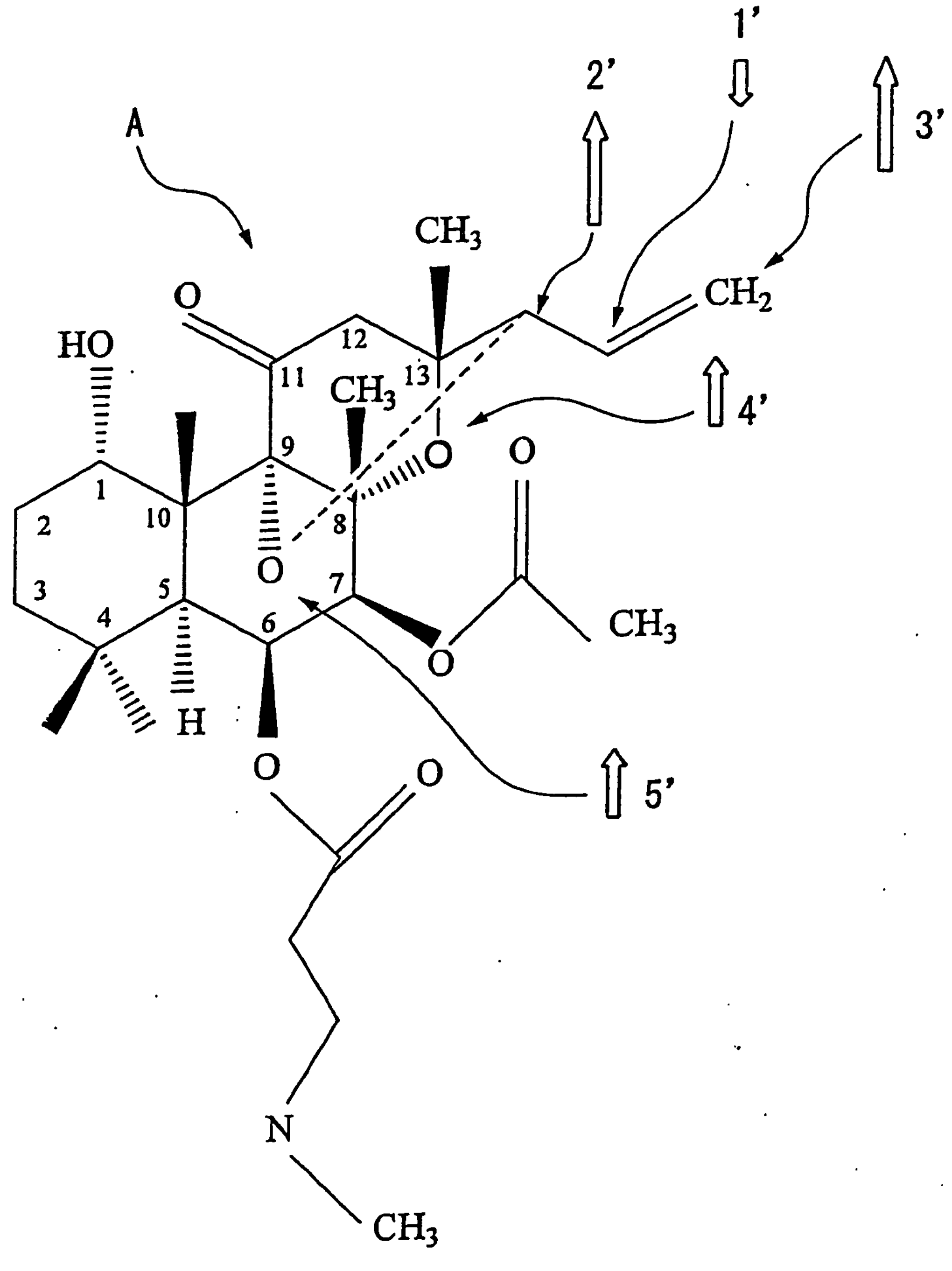

6. A magnetic drug derivative of formula:

B

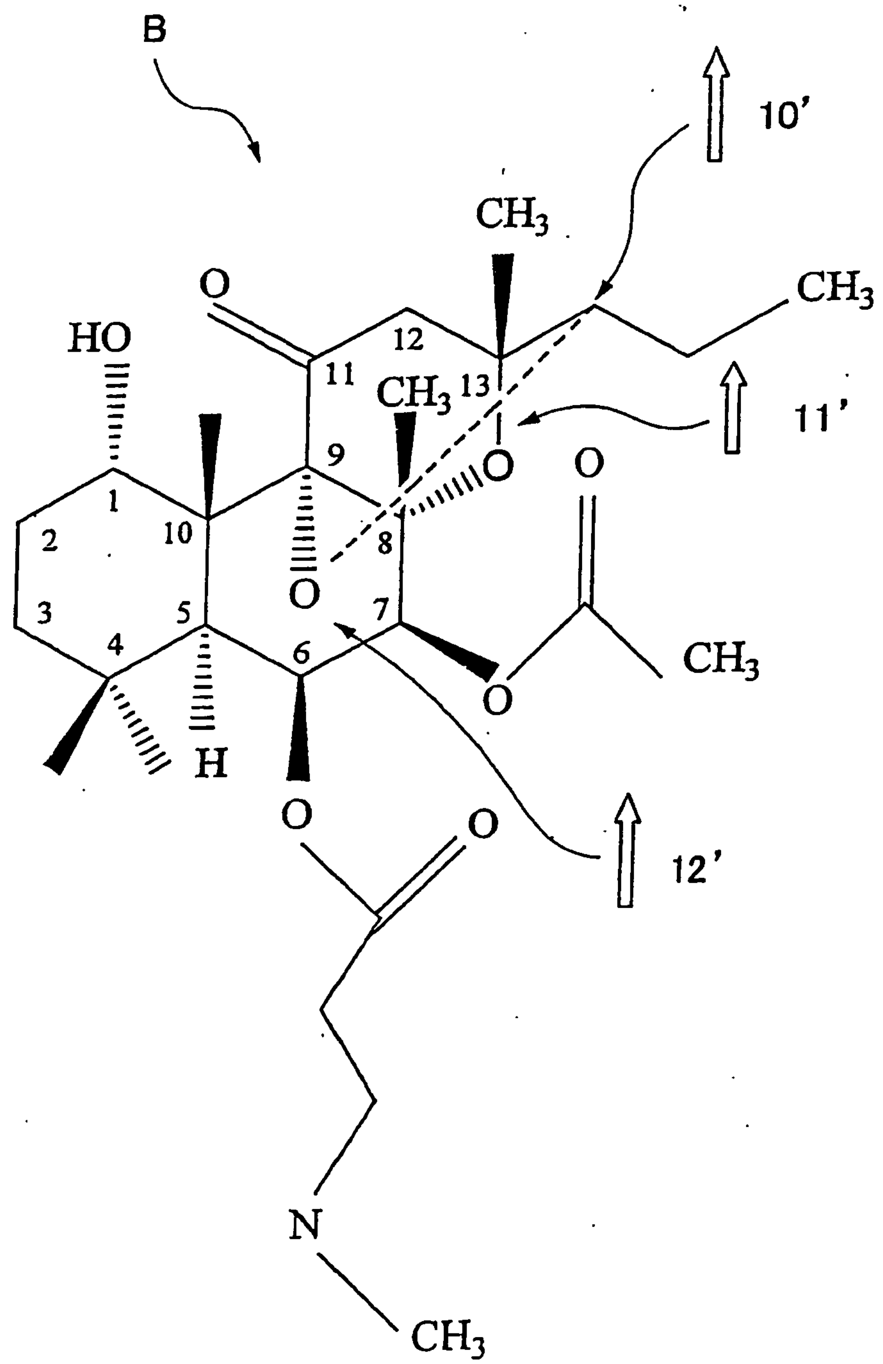

7. A magnetic drug derivative for use according to any of claims 1 to 4, or a magnetic drug derivative according to claim 5 and 6 which when administered in a body is guided to a predetermined target site using the magnetism of the magnetic drug derivative, by a drug guidance system which generates a magnetic field.

8. A method of preparing a magnetic drug derivative, comprising making a non-magnetic organic drug compound magnetic by modification of side chains and/or cross-linking between side chains without using supports made from magnetic bodies said derivative being for use in a method of treatment of male erectile dysfunction in which the derivative is guided to a target side of a body by use of the magnetism of the drug derivative itself, wherein said non-magnetic organic drug compound is a PDE5 inhibitor.

9. A method of preparing a magnetic drug derivative, comprising making a non-magnetic drug compound magnetic by modification of side chains and/or cross-linking between side chains without using supports made from magnetic bodies said derivative being for use in a method of treatment of cancer in which the derivative is guided to a target site of a body by use of the magnetism of the drug derivative itself, wherein said non-magnetic drug compound is a metal complex.

10. A method according to claims 8 or 9, comprising determining whether said magnetic drug derivative is ferromagnetic or ferrimagnetic, based on a spin-charge density distribution obtained by a numerical calculation for said magnetic drug derivative.

11. A method according to claims 8, 9 or 10, comprising determining the magnetic strength of said magnetic drug derivative, based on a spin-charge density distribution obtained by a numerical calculation for said magnetic drug derivative.

## Patentansprüche

1. Magnetisches Medikamentderivat, das durch Magnetischmachen einer nichtmagnetischen organischen Medikamentverbindung durch Modifizieren von Seitenketten und/oder Vernetzen zwischen Seitenketten ohne Verwenden von aus magnetischen Körpern bestehenden Trägem erhältlich ist, wobei das Derivat der Verwendung bei einem Verfahren zur Behandlung von Erektionsschwierigkeiten beim Mann dient, wobei das Derivat an eine Zielstelle eines Körpers mittels des Magnetismus des Medikamentderivats selbst geführt wird, wobei die nichtmagnetische organische Medikamentverbindung ein PDE5-Hemmer ist.

2. Magnetisches Medikamentderivat, das durch Magnetischmachen einer nichtmagnetischen Medikamentverbindung durch Modifizieren von Seitenketten und/oder Vernetzen zwischen Seitenketten ohne Verwenden von aus magnetischen Körpern bestehenden Trägern erhältlich ist, wobei das Derivat der Verwendung bei einem Verfahren zur Behandlung von Krebs dient, wobei das Derivat an eine Zielstelle eines Körpers mittels des Magnetismus des Medikamentderivats selbst geführt wird, wobei die nichtmagnetische Medikamentverbindung ein Metallkomplex ist.

3. Magnetisches Medikamentderivat zur Verwendung nach Anspruch 2, wobei der Metallkomplex ein cis-geometrisches Isomer mit Antikrebseigenschaften ist.

4. Magnetisches Medikamentderivat zur Verwendung nach Anspruch 3, wobei das cis-geometrische Isomer Cisplatin ist.

5. Magnetisches Medikamentderivat der Formel:

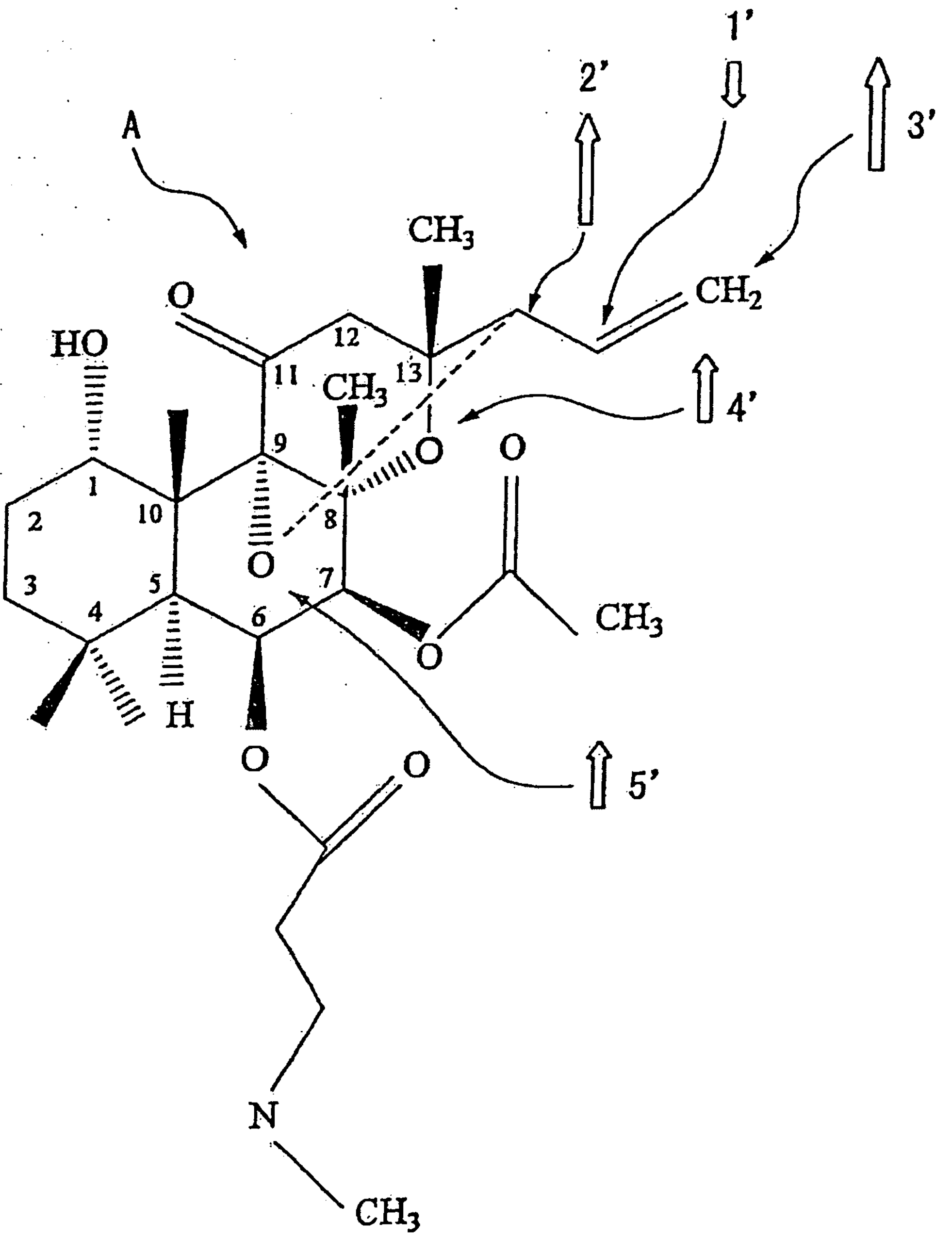

**6.** Magnetisches Medikamentderivat der Formel:

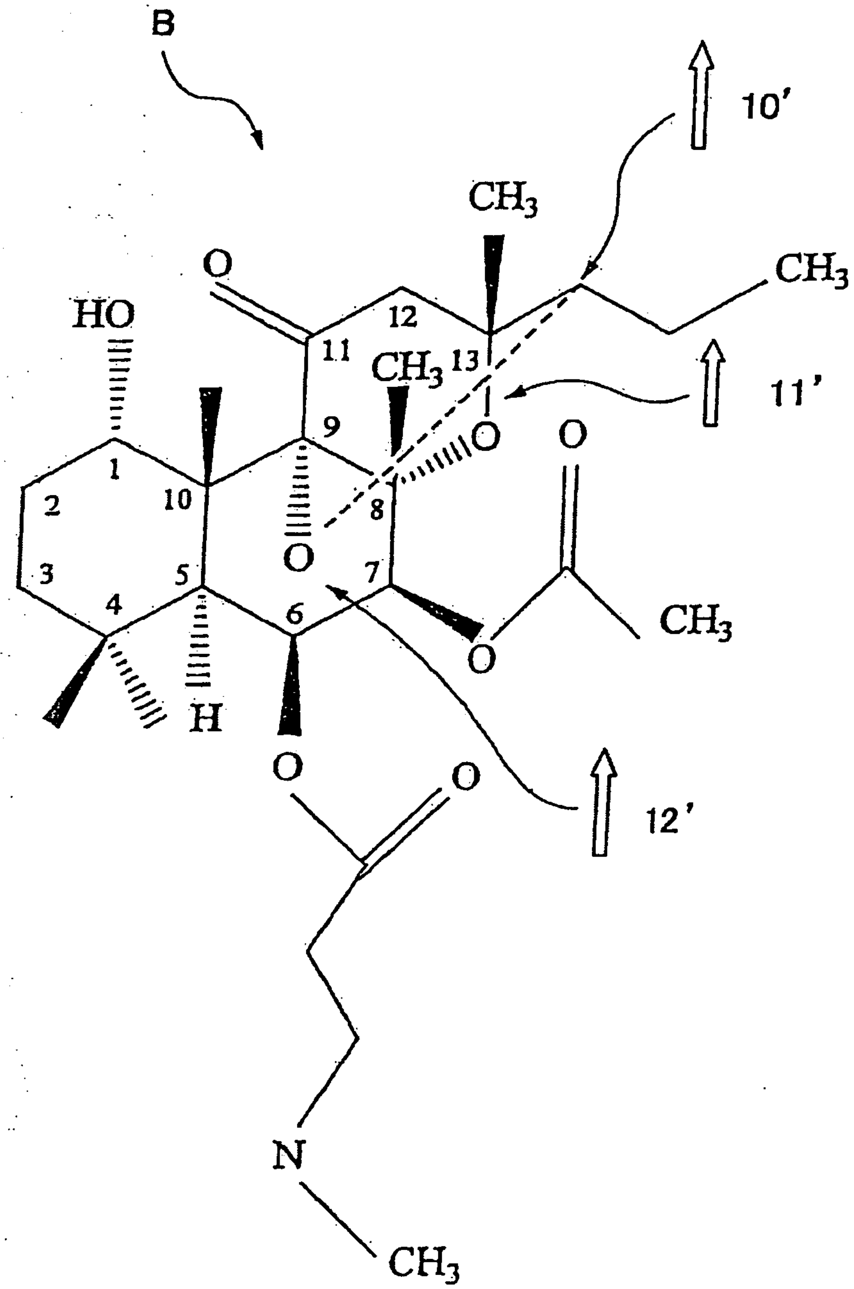

7. Magnetisches Medikamentderivat zur Verwendung nach einem der Ansprüche 1 bis 4 oder magnetisches Medikamentderivat nach Anspruch 5 und 6, das, wird es in einem Körper verabreicht, an eine vorbestimmte Zielstelle mittels des Magnetismus des magnetischen Medikamentderivats durch eine Medikamentführungssystem geführt wird, das ein magnetisches Feld erzeugt.

8. Verfahren zum Herstellen eines magnetischen Medikamentderivats, umfassend das Magnetischmachen einer nichtmagnetischen organischen Medikamentverbindung durch Modifizieren von Seitenketten und/oder Vernetzen zwischen Seitenketten ohne Verwenden von aus magnetischen Körpern bestehenden Trägern, wobei das Derivat der Verwendung bei einem Verfahren zur Behandlung von Erektionsschwierigkeiten beim Mann dient, wobei das Derivat an eine Zielstelle eines Körpers mittels des Magnetismus des Medikamentderivats selbst geführt wird, wobei die nichtmagnetische organische Medikamentverbindung ein PDE5-Hemmer ist.

9. Verfahren zum Herstellen eines magnetischen Medikamentderivats, umfassend das Magnetischmachen einer nichtmagnetischen Medikamentverbindung durch Modifizieren von Seitenketten und/oder Vernetzen zwischen Seitenketten ohne Verwenden von aus magnetischen Körpern bestehenden Trägem, wobei das Derivat der Verwendung bei einem Verfahren zur Behandlung von Krebs dient, wobei das Derivat an eine Zielstelle eines Körpers mittels des Magnetismus des Medikamentderivats selbst geführt wird, wobei die nichtmagnetische Medikamentverbindung ein Metallkomplex ist.

**10.** Verfahren nach Anspruch 8 oder 9, umfassend das Bestimmen, ob das magnetische Medikamentderivat ferromagnetisch oder ferrimagnetisch ist, auf der Basis einer Spinladungsdichteverteilung, die durch eine in Zahlen ausgedrückte Berechnung für das magnetische Medikamentderivat erhalten wird.

**11.** Verfahren nach Anspruch 8, 9 oder 10, umfassend das Bestimmen der Magnetstärke des magnetischen Medikamentderivats auf der Basis einer Spinladungsdichteverteilung, die durch eine in Zahlen ausgedrückte Berechnung für das magnetische Medikamentderivat erhalten wird.

## Revendications

**1.** Dérivé de médicament magnétique que l’on peut obtenir en magnétisant un composé médicamenteux organique non magnétique par modification des chaînes latérales et/ou réticulation entre les chaînes latérales sans utilisation de supports formés de corps magnétiques, ledit dérivé étant utilisable dans une méthode de traitement de la dysérection chez l’homme dans laquelle le dérivé est guidé vers un site cible d’un corps grâce au magnétisme du dérivé de médicament lui-même, ledit composé médicamenteux organique non magnétique étant un inhibiteur de PDE5.

**2.** Dérivé de médicament magnétique que l’on peut obtenir en magnétisant un composé médicamenteux non magnétique par modification des chaînes latérales et/ou réticulation entre les chaînes latérales sans utilisation de supports formés de corps magnétiques, ledit dérivé étant utilisable dans une méthode de traitement du cancer dans laquelle le dérivé est guidé vers un site cible d’un corps grâce au magnétisme du dérivé de médicament lui-même, ledit composé médicamenteux non magnétique étant un complexe métallique.

**3.** Dérivé de médicament magnétique à utiliser selon la revendication 2, ledit complexe métallique étant un isomère géométrique cis doté de propriétés anticancéreuses.

**4.** Dérivé de médicament magnétique à utiliser selon la revendication 3, ledit isomère géométrique cis étant le cisplatine.

**5.** Dérivé de médicament magnétique de formule:

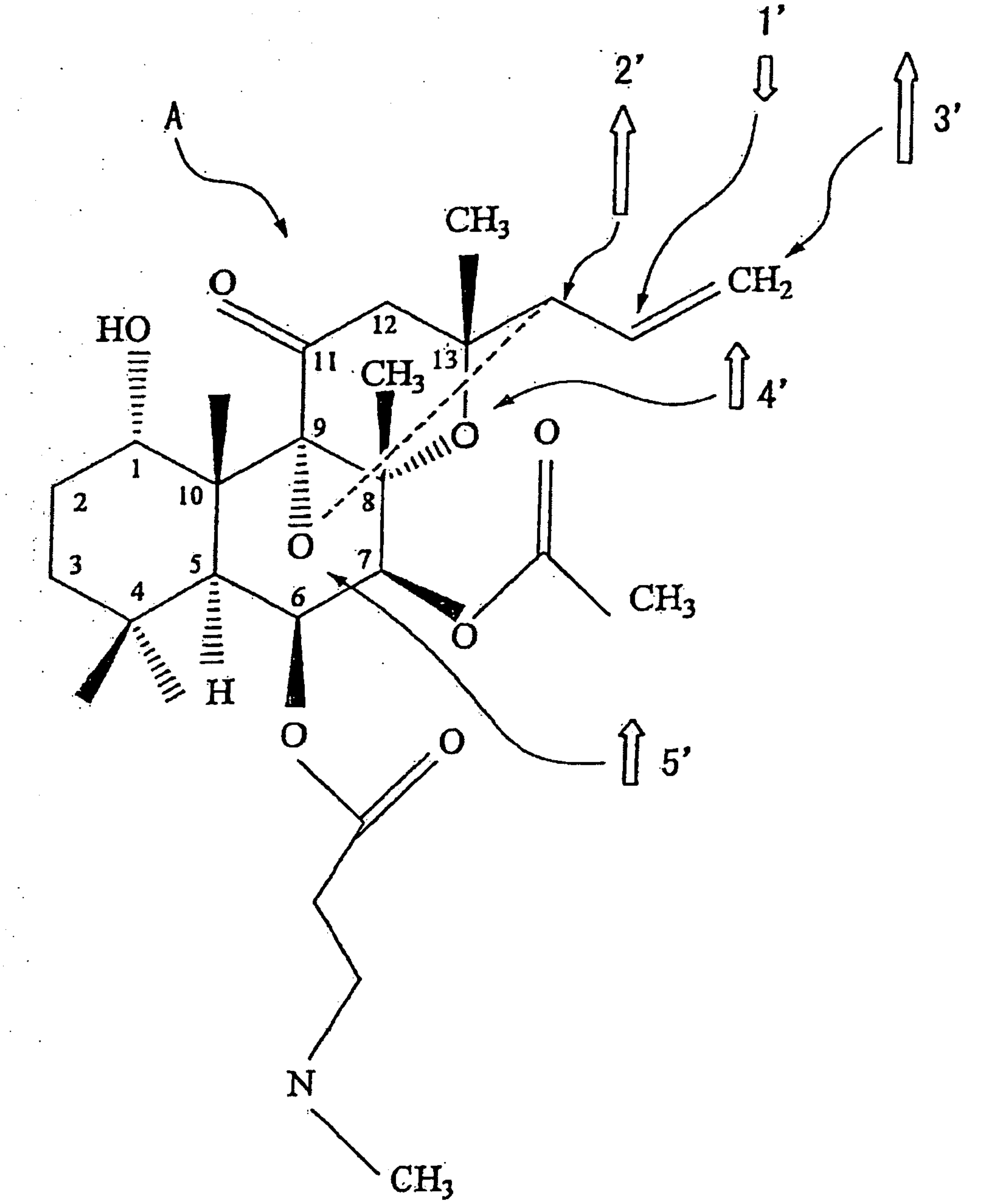

6. Dérivé de médicament magnétique de formule:

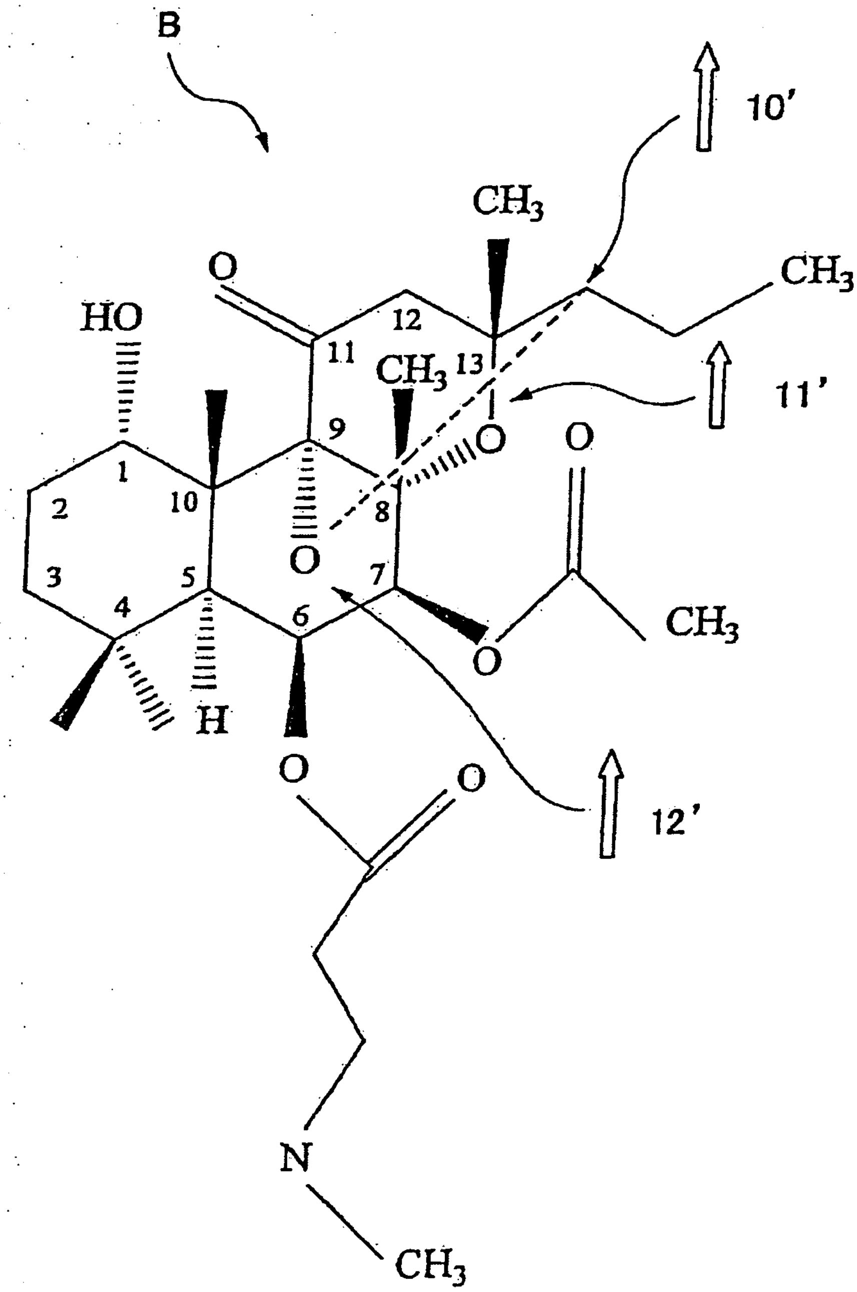

7. Dérivé de médicament magnétique à utiliser selon l'une quelconque des revendications 1 à 4, ou dérivé de médicament magnétique selon la revendication 5 et 6 qui lorsqu'il est administré dans un corps est guidé vers un site cible prédéfini grâce au magnétisme du dérivé de médicament magnétique, à l'aide d'un système de guidage de médicament qui crée un champ magnétique.

8. Procédé de préparation d'un dérivé de médicament magnétique, comprenant la magnétisation d'un composé médicamenteux organique non magnétique par modification des chaînes latérales et/ou réticulation entre les chaînes latérales sans utilisation de supports formés de corps magnétiques, ledit dérivé étant utilisable dans une méthode de traitement de la dysérection chez l'homme dans laquelle le dérivé est guidé vers un site cible d'un corps grâce au magnétisme du dérivé de médicament lui-même, ledit composé médicamenteux organique non magnétique étant un inhibiteur de PDE5.

9. Procédé de préparation d'un dérivé de médicament magnétique, comprenant la magnétisation d'un composé médicamenteux non magnétique par modification des chaînes latérales et/ou réticulation entre les chaînes latérales

sans utilisation de supports formés de corps magnétiques, ledit dérivé étant utilisable dans une méthode de traitement du cancer dans laquelle le dérivé est guidé vers un site cible d'un corps grâce au magnétisme du dérivé de médicament lui-même, ledit composé médicamenteux non magnétique étant un complexe métallique.

**10.** Procédé selon les revendications 8 ou 9, comprenant la détermination de la nature ferromagnétique ou ferrimagnétique dudit dérivé de médicament magnétique, sur la base d'une distribution des densités de charge-spin obtenue par un calcul numérique pour ledit dérivé de médicament magnétique.

**11.** Procédé selon les revendications 8, 9 ou 10, comprenant la détermination de la force magnétique dudit dérivé de médicament magnétique, sur la base d'une distribution des densités de charge-spin obtenue par un calcul numérique pour ledit dérivé de médicament magnétique.

FIG. 1

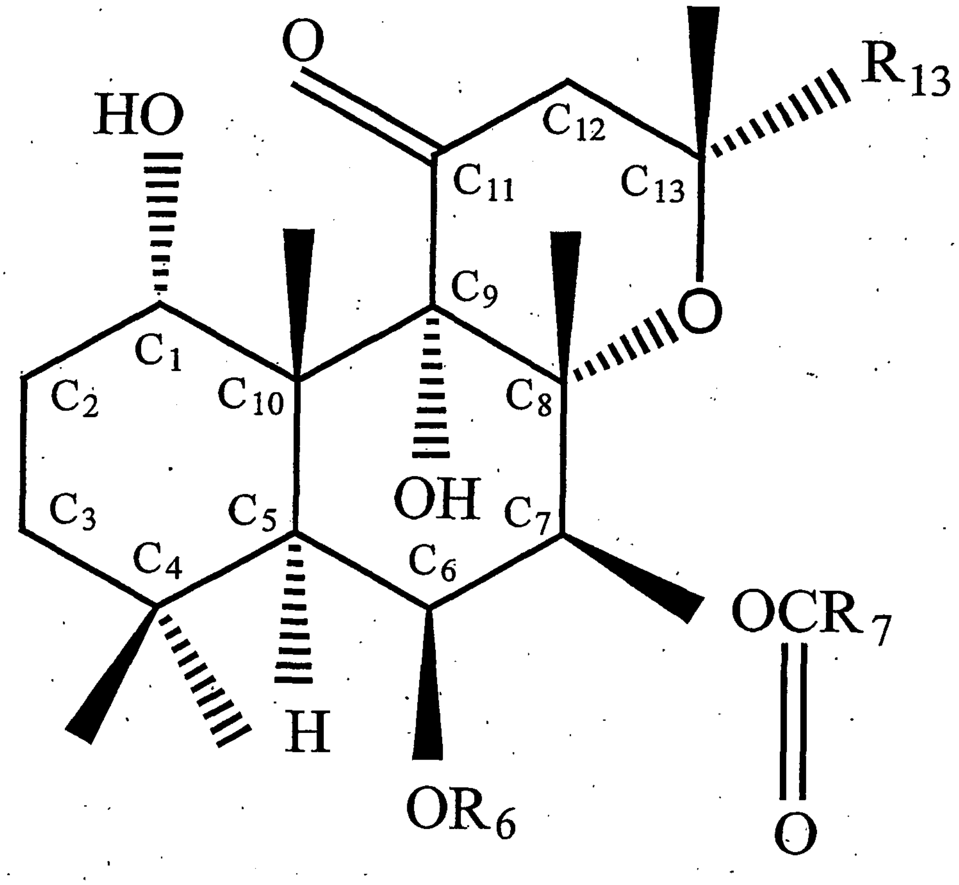

FIG. 2

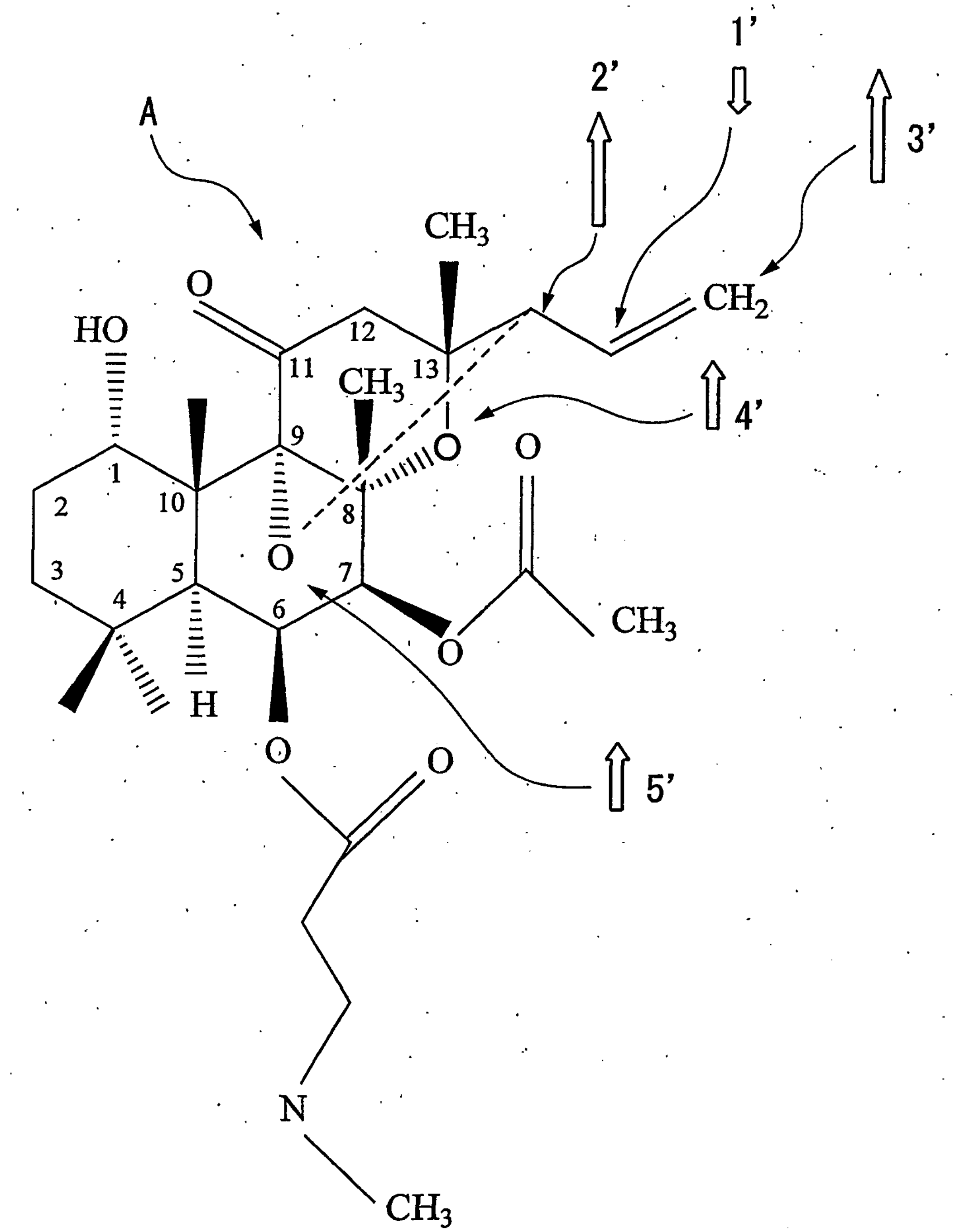

FIG. 3

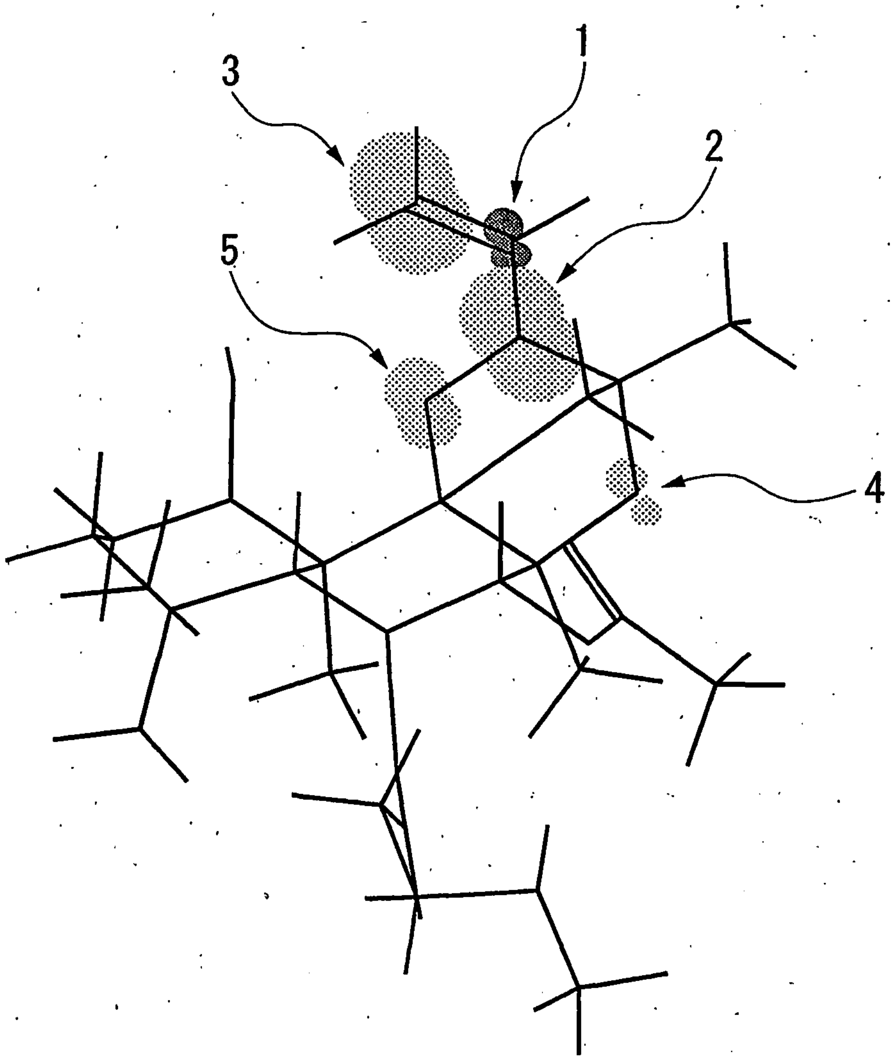

FIG. 4

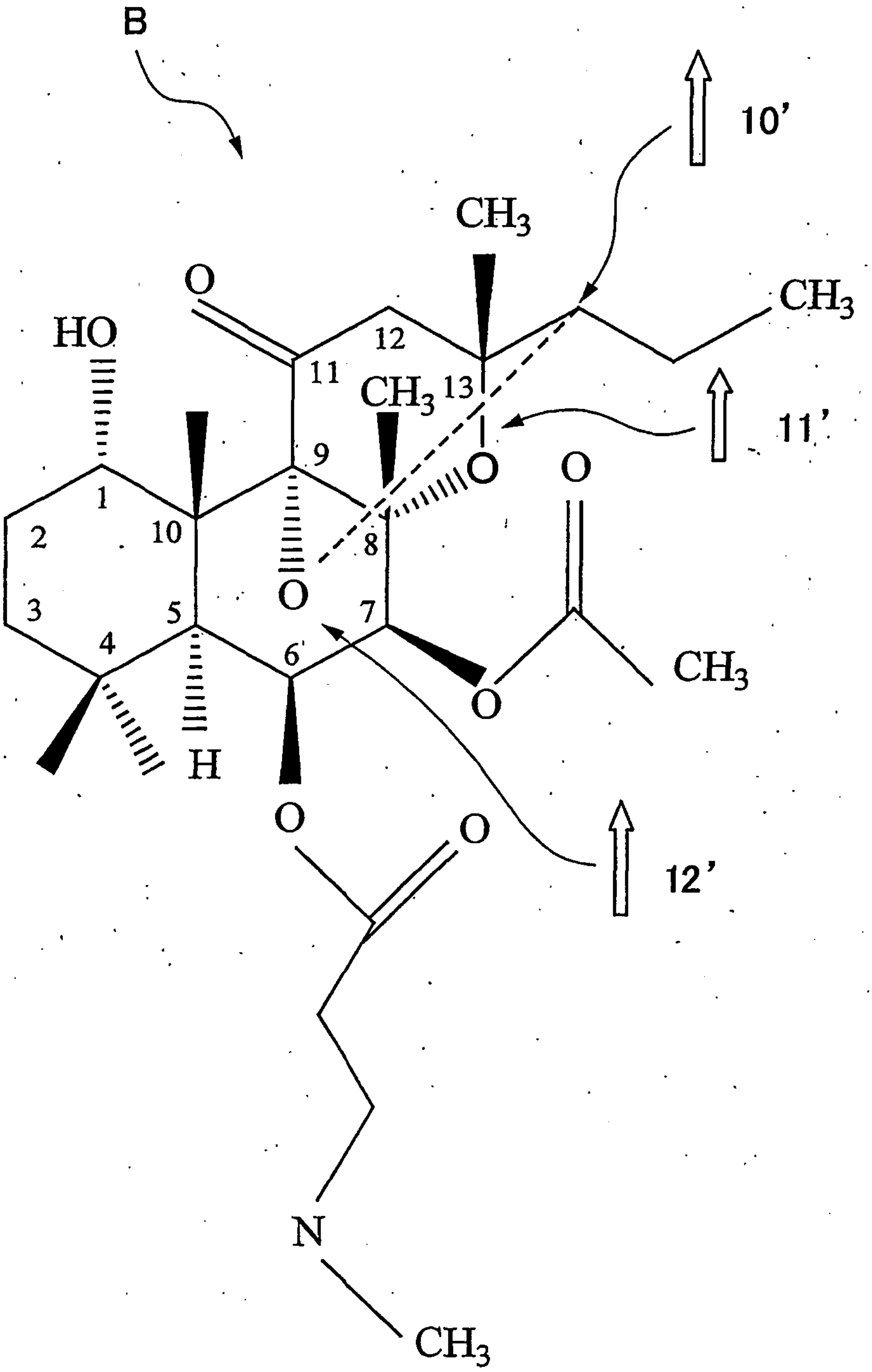

FIG. 5

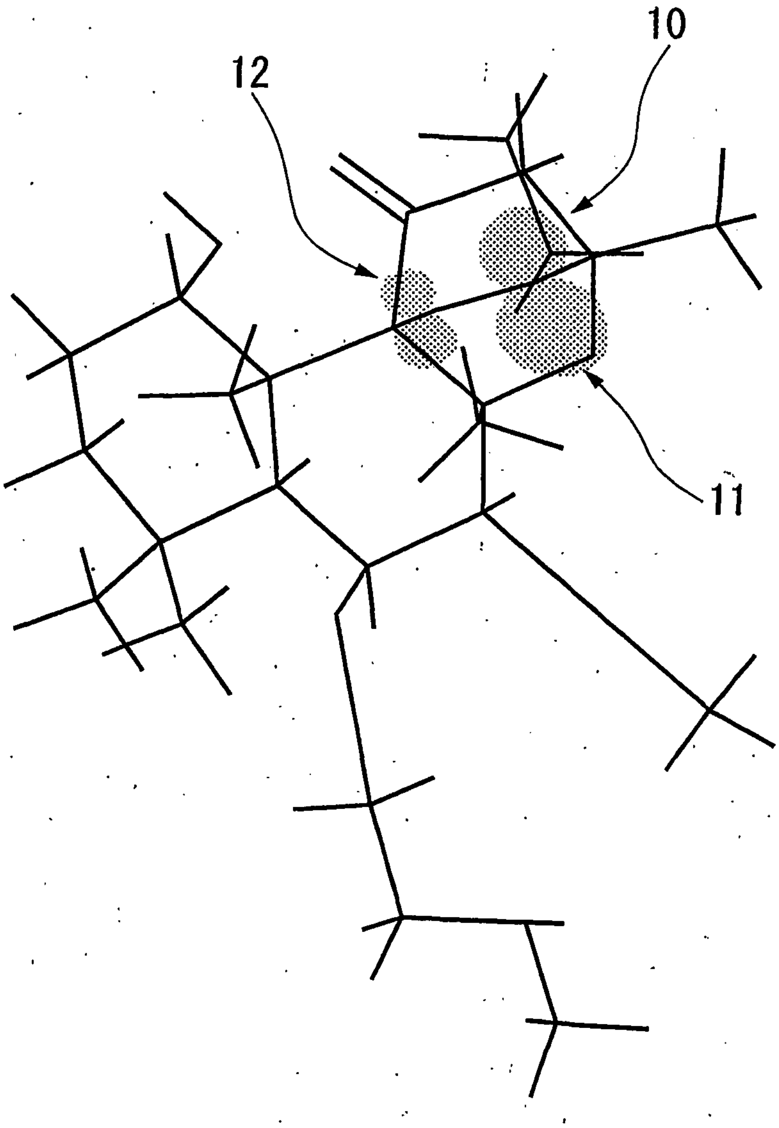

FIG. 6

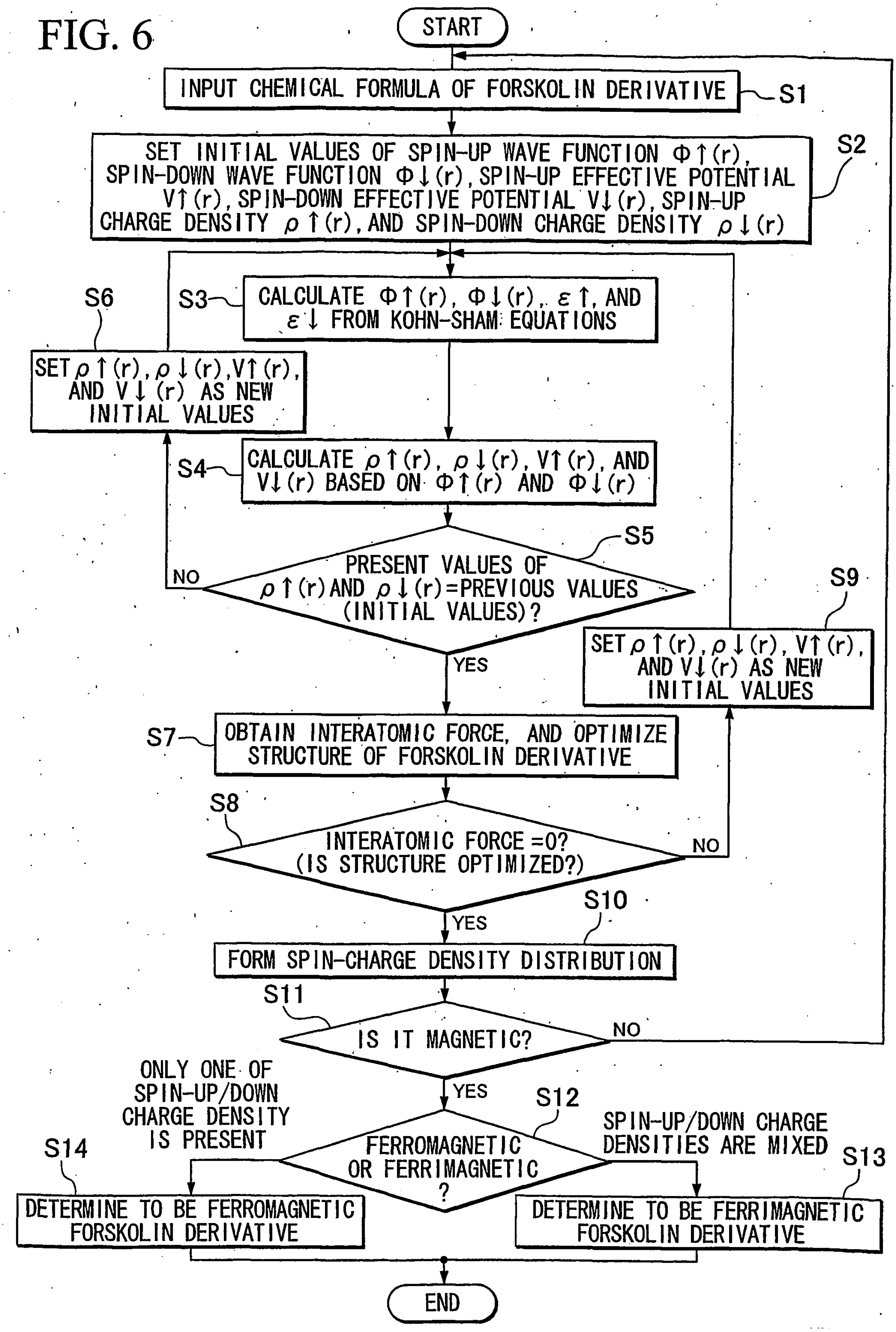
START
INPUT CHEMICAL FORMULA OF FORSKOLIN DERIVATIVE
S1
SET INITIAL VALUES OF SPIN-UP WAVE FUNCTION Φ↑(r), SPIN-DOWN WAVE FUNCTION Φ↓(r), SPIN-UP EFFECTIVE POTENTIAL V↑(r), SPIN-DOWN EFFECTIVE POTENTIAL V↓(r), SPIN-UP CHARGE DENSITY ρ↑(r), AND SPIN-DOWN CHARGE DENSITY ρ↓(r)
S2
S3
CALCULATE Φ↑(r), Φ↓(r), ε↑, AND ε↓ FROM KOHN-SHAM EQUATIONS
S6
SET ρ↑(r), ρ↓(r), V↑(r), AND V↓(r) AS NEW INITIAL VALUES
S4
CALCULATE ρ↑(r), ρ↓(r), V↑(r), AND V↓(r) BASED ON Φ↑(r) AND Φ↓(r)
S5
PRESENT VALUES OF ρ↑(r) AND ρ↓(r)=PREVIOUS VALUES (INITIAL VALUES)?
NO
YES
S9
SET ρ↑(r), ρ↓(r), V↑(r), AND V↓(r) AS NEW INITIAL VALUES
S7
OBTAIN INTERATOMIC FORCE, AND OPTIMIZE STRUCTURE OF FORSKOLIN DERIVATIVE
S8
INTERATOMIC FORCE =0? (IS STRUCTURE OPTIMIZED?)
NO
YES
S10
FORM SPIN-CHARGE DENSITY DISTRIBUTION
S11
IS IT MAGNETIC?
NO
YES
S12
FERROMAGNETIC OR FERRIMAGNETIC ?
ONLY ONE OF SPIN-UP/DOWN CHARGE DENSITY IS PRESENT
SPIN-UP/DOWN CHARGE DENSITIES ARE MIXED
S14
DETERMINE TO BE FERROMAGNETIC FORSKOLIN DERIVATIVE
S13
DETERMINE TO BE FERRIMAGNETIC FORSKOLIN DERIVATIVE
END

FIG. 7A

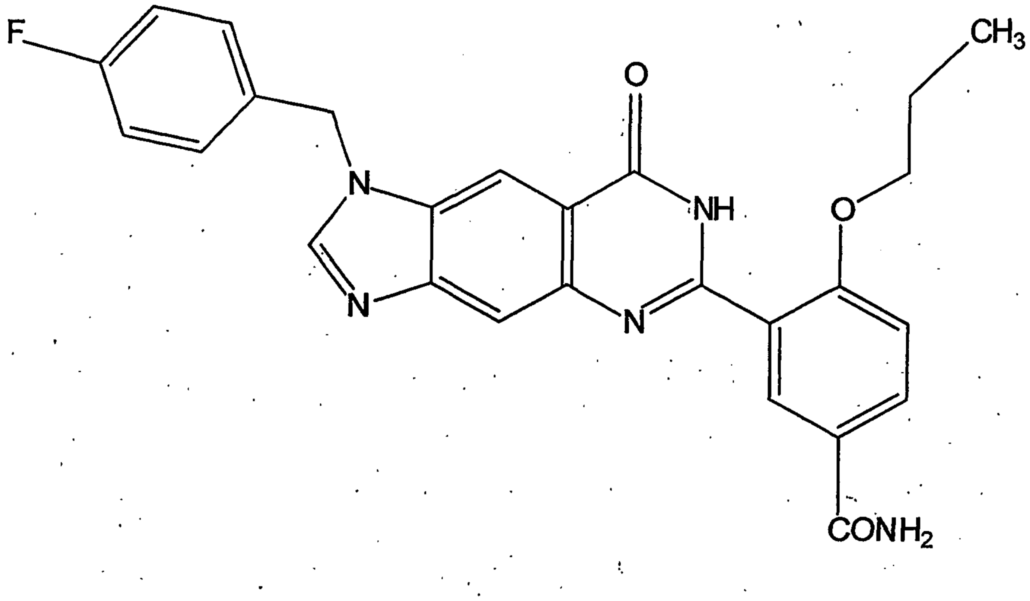

FIG. 7B

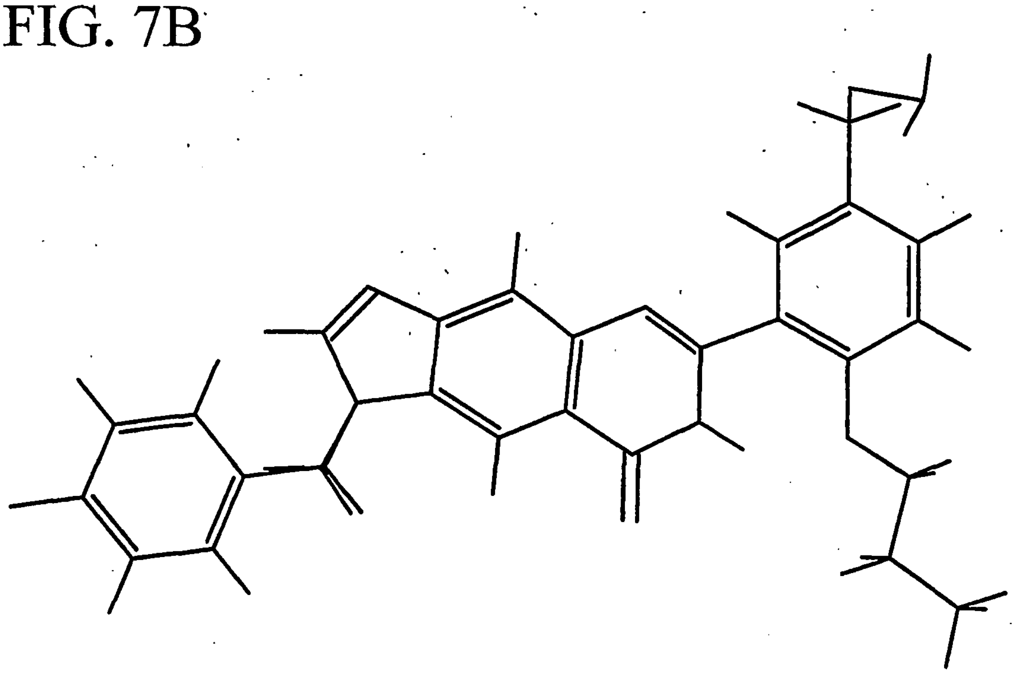

FIG. 8A

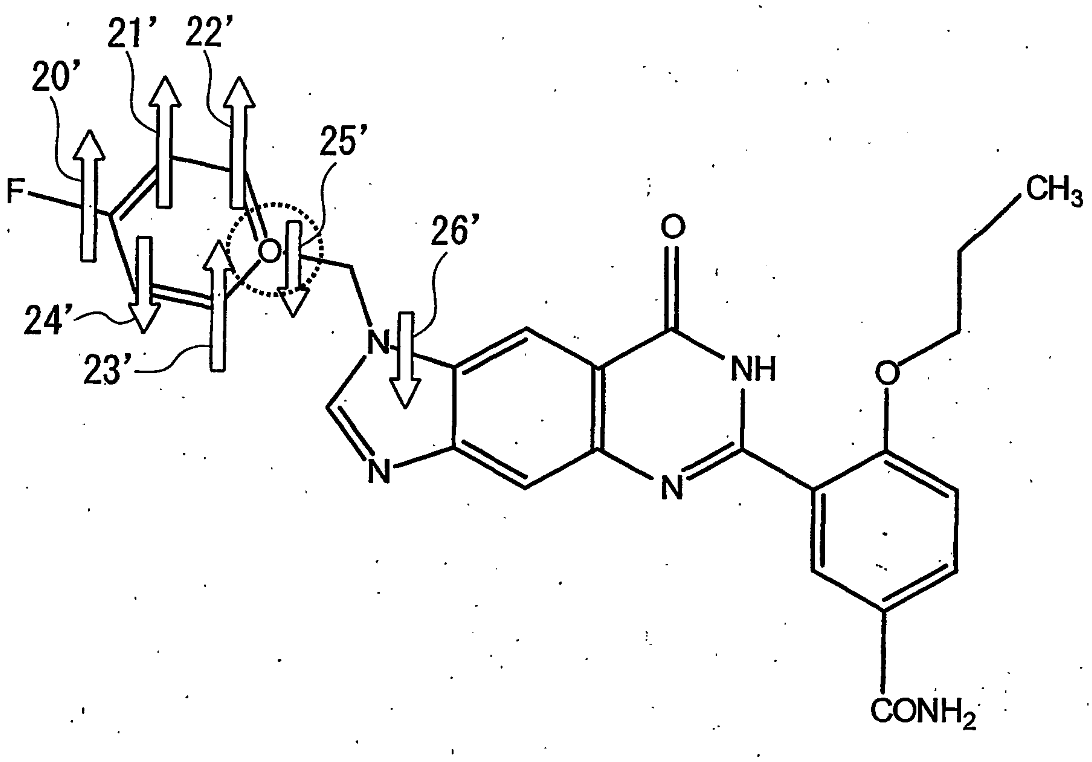

FIG. 8B

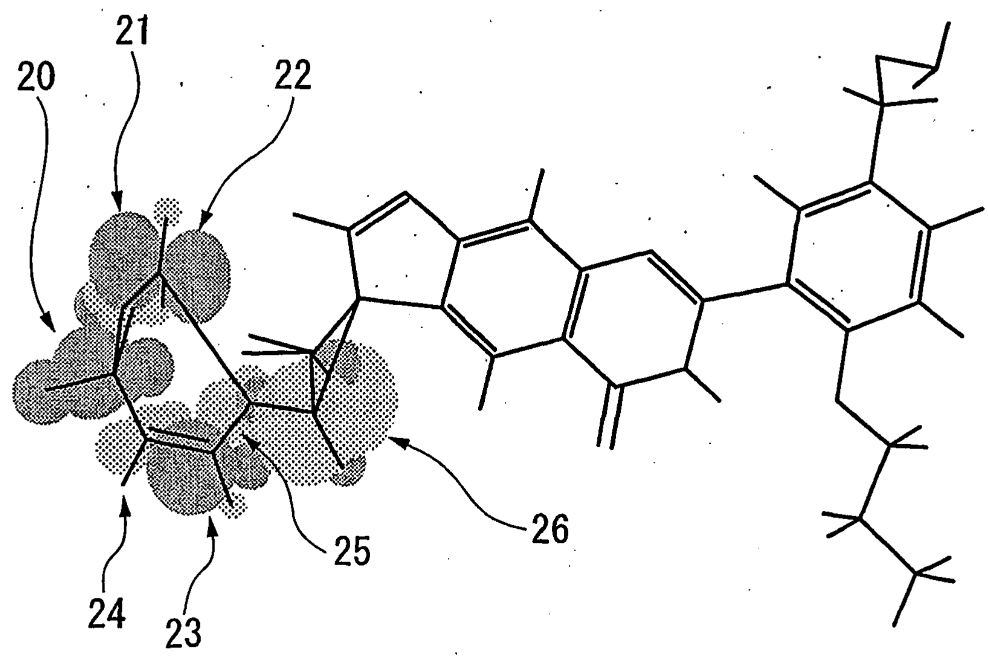

FIG. 9

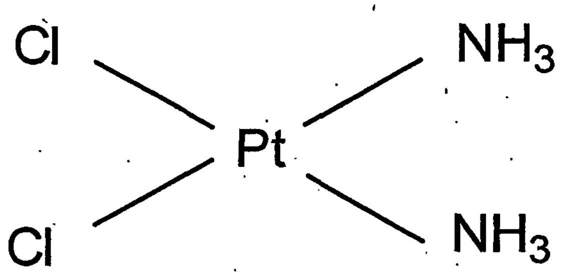

FIG. 10A

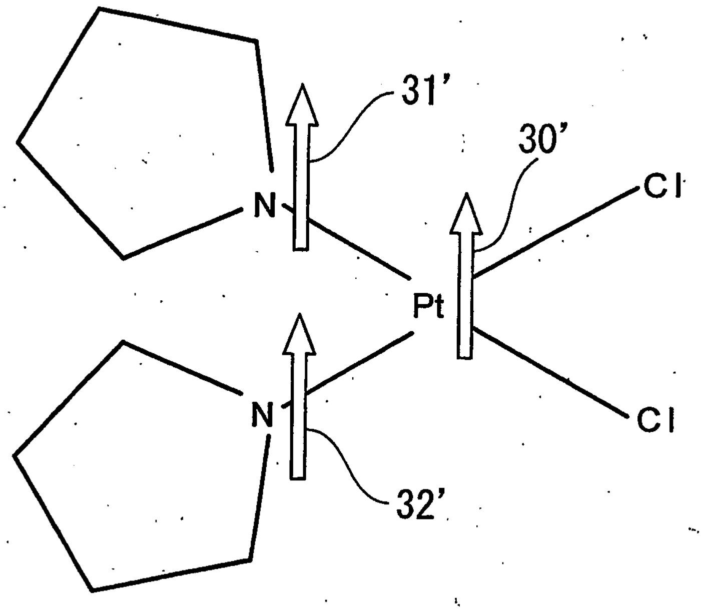


FIG. 10B

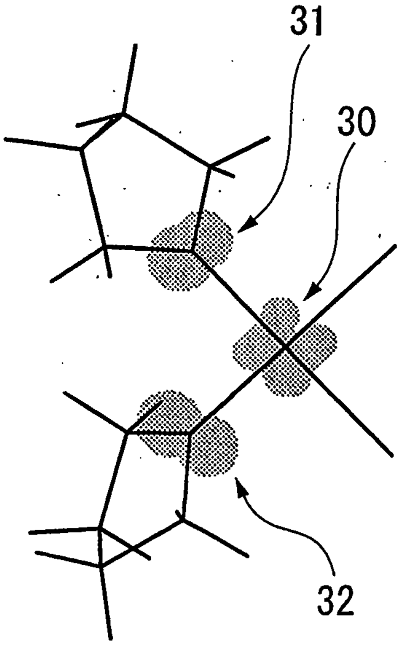

FIG. 11

| COMPOUND NAME | RELATIVE SPIN-CHARGE DENSITY |
|---|---|
| MAGNETITE ($Fe_3O_4$) PARTICLE | 1 |
| NK121 | 0.608 |
| Cis-Pt-a3 | 0.186 |
| Cis-Pt-b2 | 0.128 |
| Cis-Pt-f2 | 0.162 |
| Cis-Pt-n3 | 0.141 |
| Cis-Pd-a3 | 0.197 |
| Cis-Pd-b2 | 0.165 |
| Cis-Pd-f2 | 0.207 |
| Cis-Pd-g1 | 0.108 |
| Cis-Pd-n3 | 0.163 |
| Cis-Pd-o3 | 0.173 |
| Cis-Rh-a3 | 0.492 |
| Cis-Rh-n3 | 0.0886 |
| Cis-Rh-o3 | 0.229 |
| Cis-Ir-NK121 | 0.0709 |
| Cis-Ir-a3 | 0.0438 |
| Cis-Ir-n3 | 0.0506 |
| Cis-Ir-o3 | 0.0579 |
| Cis-Au-a3 | 0.145 |
| Cis-Ni-NK121 | 0.407 |

| COMPOUND NAME | RELATIVE SPIN-CHARGE DENSITY |
|---|---|
| Cis-Ni-a3 | 0.277 |
| Cis-Ni-b2 | 0.302 |
| Cis-Ni-c1 | 0.532 |
| Cis-Ni-d1 | 0.539 |
| Cis-Ni-e1 | 0.336 |
| Cis-Ni-f2 | 0.477 |
| Cis-Ni-g1 | 0.568 |
| Cis-Ni-n3 | 0.489 |
| Cis-Ni-o3 | 0.401 |
| Cis-Ag-a3 | 0.249 |
| Cis-Cu-a3 | 0.204 |
| Cis-Cu-o3 | 0.116 |
| Cis-Co-NK121 | 0.423 |
| Cis-Co-a3 | 0.942 |
| Cis-Co-b2 | 0.695 |
| Cis-Co-c2 | 0.949 |
| Cis-Co-d1 | 0.866 |
| Cis-Co-e1 | 0.633 |
| Cis-Co-f2 | 0.826 |
| Cis-Co-g1 | 0.715 |
| Cis-Co-n3 | 0.350 |
| Cis-Co-o3 | 0.318 |

FIG. 12

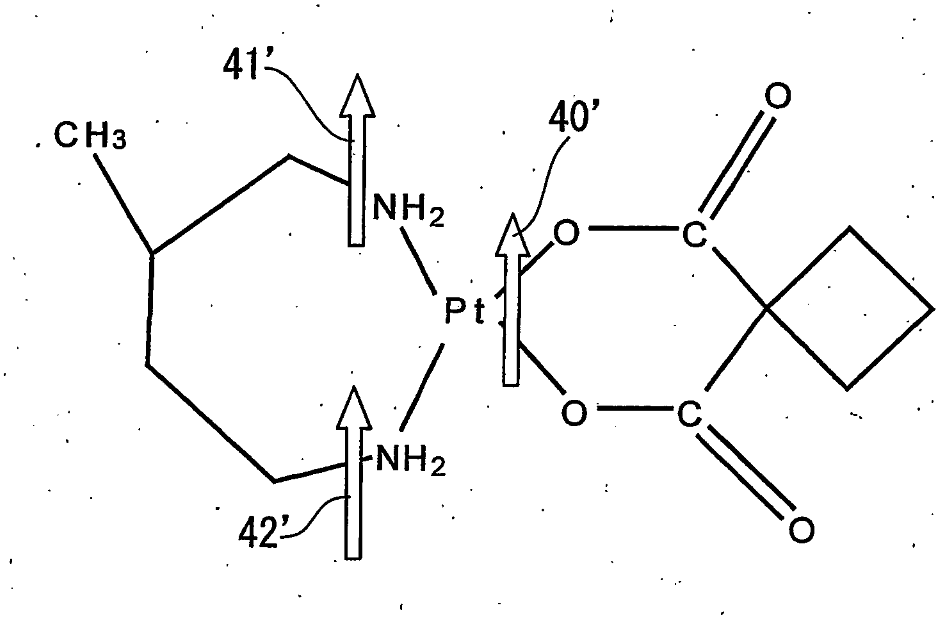

FIG. 13

REACTION 1: $cis-[PtCl_2(NH_3)_2] + H_2O \rightarrow cis-[PtCl(OH_2)(NH_3)_2]^+ + Cl^-$

REACTION 2: $cis-[PtCl(OH_2)(NH_3)_2]^+ + H_2O \rightarrow cis-[Pt(OH_2)_2(NH_3)_2]^{2+} + Cl^-$

REACTION 3: $[PtCl(dien)]^+ + H_2O \rightarrow [Pt(OH_2)_2(dien)]^{2+} + Cl^-$

$dien = (NH_2CH_2CH_2)_2NH$

FIG. 14

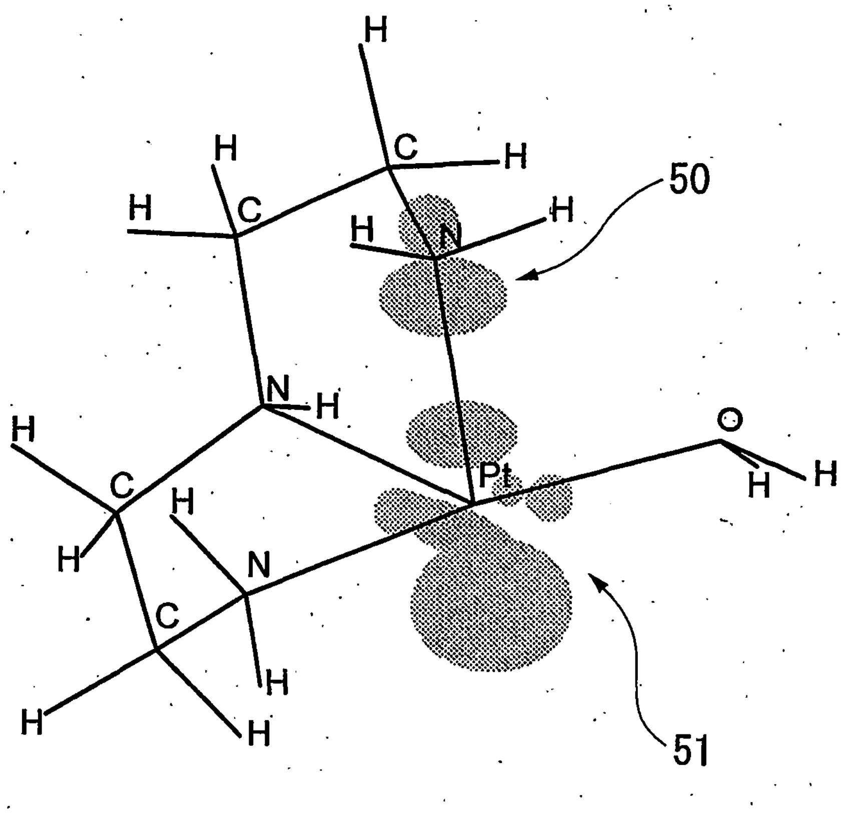

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005251190 A
- JP 2006177971 A